(19) **Europäisches Patentamt / European Patent Office / Office européen des brevets**

(11) **EP 2 161 361 B1**

(12) **EUROPEAN PATENT SPECIFICATION**

(45) Date of publication and mention of the grant of the patent:
**27.11.2013 Bulletin 2013/48**

(21) Application number: **08722384.8**

(22) Date of filing: **18.03.2008**

(51) Int Cl.:
*D04H 1/54* *(2012.01)*       *A61F 13/15* *(2006.01)*
*A61F 13/511* *(2006.01)*     *D04H 1/42* *(2012.01)*
*D02G 1/12* *(2006.01)*

(86) International application number:
**PCT/JP2008/054990**

(87) International publication number:
**WO 2009/001590 (31.12.2008 Gazette 2009/01)**

(54) **NONWOVEN FABRIC AND PROCESS FOR PRODUCING THE SAME**

VLIESSTOFF UND SEIN HERSTELLUNGSVERFAHREN

TISSU NON TISSÉ ET SON PROCESSUS DE FABRICATION

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **22.06.2007  JP 2007165642**
        **05.02.2008  JP 2008025754**

(43) Date of publication of application:
**10.03.2010  Bulletin 2010/10**

(73) Proprietor: **Uni-Charm Corporation
Ehime 799-0111 (JP)**

(72) Inventors:
• **OBA, Toru**
  **Kanonji-shi**
  **Kagawa 769-1602 (JP)**

• **MIZUTANI, Satoshi**
  **Kanonji-shi**
  **Kagawa 769-1602 (JP)**
• **ISHIKAWA, Hideyuki**
  **Kanonji-shi**
  **Kagawa 769-1602 (JP)**

(74) Representative: **Sperling, Rüdiger
Staeger & Sperling
Partnerschaftsgesellschaft
Sonnenstrasse 19
80331 München (DE)**

(56) References cited:
EP-A1- 1 022 364      EP-A2- 0 277 707
EP-A2- 0 330 212      WO-A1-01/48291
DE-U1- 29 723 320     JP-A- 2001 049 530
JP-A- 2002 030 557    JP-A- 2002 249 965
JP-A- 2003 138 430    JP-A- 2005 089 937
JP-A- 2006 124 903    JP-A- 2006 233 345

## Description

### TECHNICAL FIELD

The present invention relates to a liquid-pervious nonwoven fabric, particularly to such a nonwoven fabric suitably used as a liquid-pervious top sheet in a liquid-absorbent article such as a disposable diaper or sanitary napkin and to a method for making the same.

### BACKGROUND ART

Conventionally it has been required for liquid-pervious topsheets used to cover liquid-absorbent cores such as bodily fluid absorbent cores in disposable diapers to spot-absorb bodily fluid without spreading it and to transfer it quickly into the cores. The invention relating to sanitary material having a high water-permeability, for example, disclosed in JP 1998-5275 A has proposed the top sheet of the type described herein. The top sheet disclosed JP 1998-5275 A is obtained by spraying a mixture of polyether compound and polyether modified silicone to modify the top sheet hydrophilic onto a crimped or crimp-free spun bond nonwoven fabric made of polypropylene so that an initial water permeation rate of 0.25 sec or less.

### DISCLOSURE OF THE INVENTION

### PROBLEM TO BE SOLVED BY THE INVENTION

An embodiment of the top sheet disclosed in JP 1998-5275 A is the top sheet formed of a spun bond nonwoven fabric of polypropylene sprayed with an agent modifying the top sheet to be hydrophilic. Most of fibers are placed one upon another between an upper surface and a lower surface of the nonwoven fabric and extend in parallel to these upper and lower surfaces. As a result, discharged bodily fluids can be spot-absorbed so far as discharged bodily fluids is relatively small. However, bodily fluids will horizontally spread and it will become inevitably difficult for the top sheet to spot-absorb bodily fluids when a relatively large amount of bodily fluids is discharged. Furthermore, in the case of such known topsheet, a time necessary for bodily fluids to permeate the top sheet becomes unacceptably long as viscosity of bodily fluids increases.

In view of the problem left unsolved behind by the prior art as has been described above, it is an object of the present invention to provide a nonwoven fabric and a method for making the same improved to solve the problem.

### MEASURE TO SOLVE THE PROBLEM

The object set forth above is achieved by a first aspect of the present invention relating to a liquid-pervious nonwoven fabric, on one hand, and by a second aspect of the present invention relating to a method for making the nonwoven fabric, on the other hand.

The first aspect of the invention relates to a liquid-pervious nonwoven fabric having a basis weight in a range of 10 to 200 g/m<2>, the liquid-pervious nonwoven fabric further having a first direction, a second direction orthogonal to the first direction and a thickness direction in orthogonal relationship one to another, the nonwoven fabric comprising core-sheath type composite fibers of 100 to 30% by weight as composite fibers and thermoplastic synthetic fibers of 0 to 70% by weight as blending fibers for the composite fibers, the core-sheath type composite fibers comprising core and sheath components in concentric relationship with each other wherein a thermoplastic synthetic resin forming the sheath component has a fusion point lower than a fusion point of a thermoplastic synthetic resin forming the core component.

The nonwoven fabric according to the first aspect of the invention further comprising: the composite fiber has a fineness of 1 to 17 dtex and a fiber length of 10 to 150 mm wherein the composite fibers extend in the first direction with curvatures repeated in the thickness direction as viewed in a cut surface of the nonwoven fabric taken in parallel to the first direction and extends in the thickness direction as viewed in a cut surface of the nonwoven fabric taken in parallel to the second direction; the composite fibers intersect themselves and/or with the blending fibers and, at respective points of intersection, the composite fibers are fused together with themselves and/or with the blending fibers as the low fusion point resin is fused; and when the nonwoven fabric is placed in a horizontal plane, some of the composite fibers and the blending fibers intersect, in the cut surface of the nonwoven fabric taken in parallel to the second direction, a vertical line with respect to the horizontal plane at acute angles inclusive of 90[deg.] and some of the composite fibers and the blending fibers intersect the vertical line at obtuse angles larger than 90[deg.] so that an average fiber angle corresponding to an average value of the acute intersection angles may be 75[deg.] or less and wherein said nonwoven fabric has upper and lower surfaces opposed to each other in said Thickness direction (TD) and said upper surface is formed with a plurality of crests extending in parallel to said first direction and a plurality of troughs each extending in said first direction between each pair of adjacent said crests.

According to one embodiment of the first aspect of the invention, the composite fibers contain among them spirally crimped thermoplastic synthetic fibers at content up to 50% by weight.

According to another embodiment of the first aspect of the invention, at least one of hydrophilic blending natural fibers and hydrophilic blending semi-synthetic fibers are contained in the nonwoven fabric up to 10% by weight with respect to total weight of the nonwoven fabric.

According to still another embodiment of the first aspect of the invention, one of the composite fibers and the blending thermoplastic synthetic fibers have its surface modified to be hydrophilic.

According to an alternatively embodiment of the first aspect of the invention, with the lower surface of the nonwoven fabric placed in the horizontal plane, the average fiber angle defined between the vertical line extending through an apex of the crest and the composite fibers and/or the blending fibers are 75[deg.] or less.

According to an alternatively another embodiment of the first aspect of the invention, the nonwoven fabric is used as a top sheet in a sanitary napkin.

The second aspect of the invention relates to a method for making a liquid-pervious nonwoven fabric having a basis weight in a range of 10 to 200 g/m<2>, the liquid-pervious nonwoven fabric further having a mechanical direction and a cross direction in orthogonal relationship to each other and including core-sheath type composite fibers of 100 to 30% by weight as composite fibers, wherein the core-sheath type composite fibers comprising core and sheath components in concentric relationship with each other and a thermoplastic synthetic resin forming the sheath component has a fusion point lower than a fusion point of a thermoplastic synthetic resin forming the core component, the method comprising the steps as follow:

a. forming the core-sheath type composite fibers followed by obtaining a tow from a plurality of the core-sheath type composite fibers and then stretching the tow;
b. mechanically crimping the tow having been stretched in the step (a) so as to repeat curvatures in a longitudinal direction of the tow;
c. subjecting the tow having been crimped in the step (b) to an annealing treatment;
d. cutting the tow having been subjected to the annealing treatment in the step (c) into a length of 10 to 150 mm so as to obtain an assembly of the composite fibers in the form of staples;
e. fibrillating the assembly of the composite fibers through a card to obtain a web comprising the composite fibers and having a desired basis weight;
f. heating the web to fuse the resin of low fusion point and thereby fusing together the composite fibers in the web one at intersection points thereof; and
g. cooling the web after the step (f):

wherein
the method further including between said step(e) and said step(f)a step of preheating said web, after which said composite fibers have been fused together before said web is conveyed to said step (f);
said step of preheating includes a sub-step of emitting a jet of heated and pressurized air from a plurality of individual nozzles arranged in said cross direction to said web conveyed on support means in said machine direction (MD) to form said web with a plurality of crests parallel extending in said machine direction (MD) and a plurality of troughs each defined between a pair of adjacent said crests and extending in said machine direction (MD); and said annealing treatment in said step (c) is carried out at a temperature between the fusion temperature of said low fusion point resin forming said sheath components and a temperature 20°C lower than said fusion temperature.

According to one embodiment of the second aspect of the invention, the method further includes a step of arranging a plurality of the cards in the machine direction followed by placing the webs obtained from the respective cards one upon another to form a laminate web so as to be treated as the web in a step subsequent to the step (f).

According to another embodiment of the second aspect of the invention, the method further includes, between the step (e) and the step (f), a step of preheating the web after the composite fibers have been fused together at the intersection points thereof and before the web is conveyed to the step (f).

According to another embodiment of the second aspect of the invention, the step (f) includes a sub-step of compressing the web in the thickness direction using compressed air or mechanical means to increase a density of the web and a sub-step of fusing the composite fibers in the web together at the intersection points of the composite fibers.

According to still another embodiment of the second aspect of the invention, the step of preheating includes a sub-step of emitting a jet of heated and pressurized air from a plurality of individual nozzles arranged in the cross direction to the web being conveyed on support means in the machine direction to form the web with a plurality of crests parallel extending in the machine direction and a plurality of troughs each defined between a pair of the adjacent crests and extending in the machine direction.

According to yet another embodiment of the second aspect of the invention, the step (e) includes a sub-step of adding thermoplastic synthetic fibers having latent crimps as blending fibers to the composite fibers so as to occupy 0 to 50% by weight with respect to total weight of the nonwoven fabric.

According to further another embodiment of the second aspect of the invention, a ratio of thickness T of the nonwoven fabric measured in a region inclusive of an apex of the crest in a cut surface of the nonwoven fabric taken in parallel to the cross direction to a width W of the crest measured at a level corresponding to 1/2 of the thickness T is in a range of 0.55 to 1.00.

According to an alternatively embodiment of the second aspect of the invention, the step (b) comprises a step of feeding the tow into a box-type crimper so that the composite fibers may be mechanically crimped in zigzags at a rate of 10 to 35 crimps/25 mm.

According to alternativelystill another embodiment of the second aspect of the invention, the composite fibers intersect, in the cut surface of the nonwoven fabric taken in parallel to the cross direction, a vertical line with respect to the horizontal plane at acute angles inclusive of 90[deg.] and some of the composite fibers and the blending fibers intersect the vertical line at obtuse angles  larger than 90[deg.] so that an average fiber angle corresponding to an average value of the acute intersection angles may be 75[deg.] or less.

According to alternatively yet another embodiment of the second aspect of the invention, the composite fibers and the thermoplastic synthetic fibers used as the blending fibers intersect, in the cut surface of the nonwoven fabric taken in parallel to the cross direction, the vertical line with respect to the horizontal plane at acute angles inclusive of 90[deg.] and some of the composite fibers and the blending fibers intersect the vertical line at obtuse angles larger than 90[deg.] so that an average fiber angle corresponding to an average value of the acute intersection angles may be 75 [deg.] or less.

According to alternatively further another embodiment of the second aspect of the invention, the step (e) comprises a sub-step of blending at least one of hydrophilic natural fibers and hydrophilic semi-synthetic fibers in the nonwoven fabric so that a content of the hydrophilic natural fibers or the hydrophilic semi-synthetic fibers may be 0 to 10% by weight with respect to total weight of the nonwoven fabric.

EFFECT OF THE INVENTION

The nonwoven fabric according to the first aspect of this invention primarily comprises the core-sheath type composite fibers and the low fusion point resin forming the sheath component of the composite fibers may be fused together at the intersection points thereof whereby to provide the nonwoven fabric with desired high strength. These composite fibers intersect the vertical line with respect to the horizontal plane on which the nonwoven fabric is placed at an average fiber angles of 75[deg.] or less as measured in the cut surface of the nonwoven fabric taken in parallel to the cross direction of the nonwoven fabric. In other words, these composite fibers extend primarily in the thickness direction of the nonwoven fabric and thereby ensure that bodily fluids can be rapidly  transferred downward along the composite fibers in the thickness direction. In this way, bodily fluids can spot-permeate the nonwoven fabric.

The method for making the nonwoven fabric according to the second aspect of the invention includes the step of mechanically crimping the tow so that the composite fibers thereby obtained repeat curvatures in the longitudinal direction. Consequentially, the web obtained by carding these composite fibers extends in the machine direction with the repetitive curvatures in the thickness direction of the web. The nonwoven fabric obtained from the web can be improved in its tensile strength by fusing the composite fibers together at the intersection points thereof. So far as the core component is not fused even when the sheath component is fused, there is no possibility that a dimension of the web might be significantly changed in the course of fusing the sheath component and it is assured to obtain the adequately bulky nonwoven fabric from the composite fibers.

BRIEF DESCRIPTION OF THE DRAWINGS

[FIG. 1] Fig. 1 is a perspective view of a nonwoven fabric according to the present invention.
[FIG. 2] Fig. 2 is a schematic diagram exemplarily illustrating a production process for carrying out a method according to the present invention.
[FIG. 3] Fig. 3 is a diagram partially showing a production process for carrying out the method according to one embodiment of the invention differing from an embodiment illustrated in Fig. 2.
[FIG. 4] Fig. 4 is a perspective view of the nonwoven fabric having a configuration differing from that shown in Fig. 1.
[FIG. 5] Fig. 5 is a schematic diagram showing the production process for carrying out the method according to one embodiment of the invention differing from the embodiments shown in Figs. 2 and 3, respectively.

[FIG. 6] Fig. 6 is a schematic diagram partially showing a member used in a suction drum.

[FIG. 7] Fig. 7 is a schematic diagram partially showing the production process for carrying out the method according to one embodiment differing from the embodiments showing in Figs. 2, 3 and 5, respectively.

[FIG. 8] Fig. 8 is a diagram exemplarily showing layout of individual nozzles.

[FIG. 9] Fig. 9 is a sectional view of the nonwoven fabric, indicating height and width of each crest thereof.

[FIG. 10] Fig. 10 is a partially cutaway perspective view of a sanitary napkin as an example of articles using the nonwoven fabric.

[FIG. 11] Fig. 11 is a diagram showing how to measure an average fiber angle in a nonwoven fabric according to one embodiment.

[FIG. 12] Fig. 12 is a diagram showing how to measure the average fiber angle in a nonwoven fabric shown in Fig. 11.

[FIG. 13] Fig. 13 is an exemplary cut surface of the nonwoven fabric of Fig. 11 taken in parallel to the machine direction.

[FIG. 14] Fig. 14 is a diagram illustrating how to measure the average fiber angle in the nonwoven fabric according to one embodiment differing from the embodiment shown in Fig. 11.

[FIG. 15] Fig. 15 is a diagram illustrating how to measure the average fiber angle in the nonwoven fabric shown in Fig. 14.

[FIG. 16] Fig. 16 is an exemplary cut surface of the nonwoven fabric of Fig. 14 taken in parallel to the machine direction.

[FIG. 17] Fig. 17 is a diagram showing how to measure the average fiber angle in the nonwoven fabric according to one comparative embodiment.

[FIG. 18] Fig. 18 is a diagram showing how to measure the average fiber angle in the nonwoven fabric shown by Fig. 17.

[FIG. 19] Fig. 19 is an exemplary cut surface of the nonwoven fabric of Fig. 17 taken in parallel to the machine direction.

[FIG. 20] Fig. 20 is a graphic diagram plotting the measurement result of temperature changing rate.

[FIG. 21] Fig. 21 is a graphic diagram plotting T/W versus average fiber angle.

[IDENTIFICATION OF REFERENCE NUMERALS USED IN THE DRAWINGS]

| 1 | nonwoven fabric |
| 2 | composite fiber |
| 104, 105, 106, 107 | step (f) |
| 112 | blending fibers |
| 201 | nonwoven fabric |
| 202 | crest |
| 203 | trough |
| 210 | step of preheating (molding means) |
| 211 | supporting member (suction drum) |
| MD | machine direction |
| CD | cross direction |
| TD | thickness direction |

DETAILED DESCRIPTION OF THE PREFERRED EMBODIMENTS

Details of a nonwoven fabric and a method for making the same will be more fully understood from the description given hereunder with reference to the accompanying drawings.

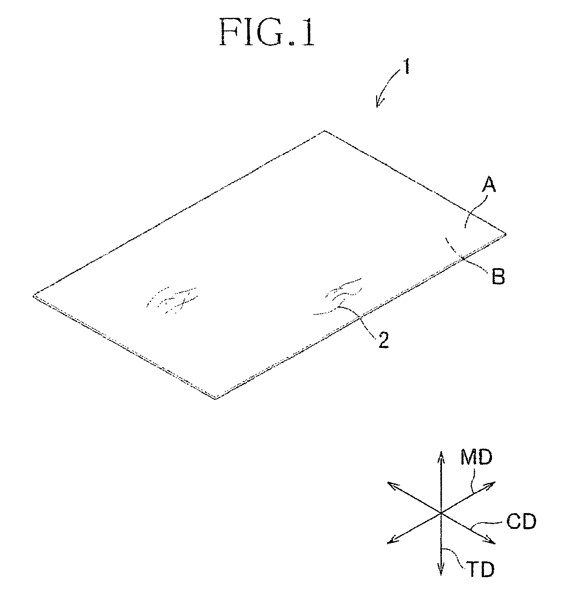

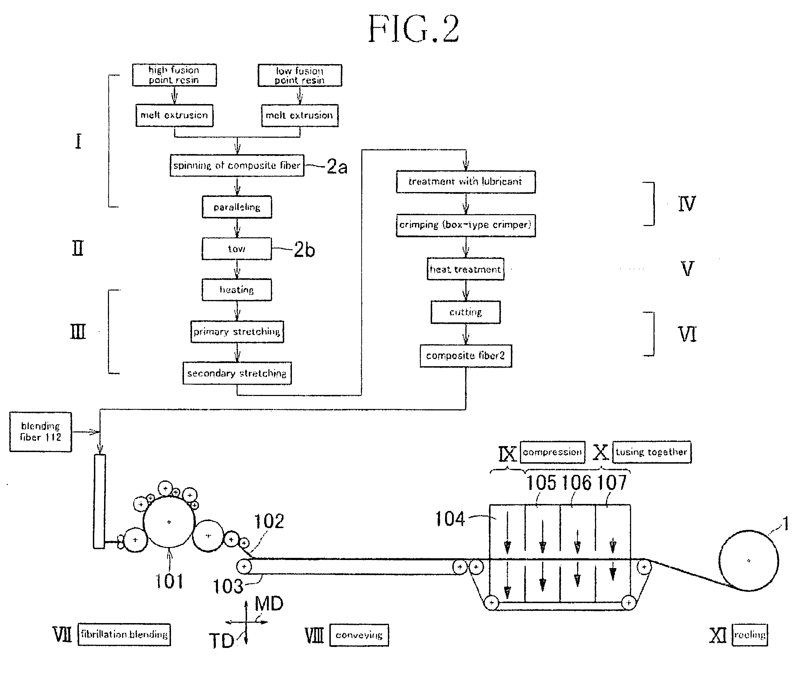

Fig. 1 is a perspective view of a nonwoven fabric 1 and Fig. 2 is a schematic diagram showing a production process for the nonwoven fabric 1. The nonwoven fabric 1 has a first direction (machine direction) MD extending in parallel to a direction in which the production process illustrated in Fig. 2 progresses, a second direction (cross direction) CD extending transversely with respect to the production process, i.e., orthogonally to the first direction MD, and a thickness direction TD extending orthogonally to these two directions MD, CD. Upper and lower surfaces of the nonwoven fabric 1 as viewed in the thickness direction are designated by A and B, respectively. The nonwoven fabric 1 comprises core-sheath type composite fibers 2 of 100 to 30% by weight and has a basis weight of 10 to 200 g/m$^2$ and a thickness of 0.3 to 15 mm. The nonwoven fabric 1 is significantly liquid-pervious for the reason that the composite fibers 2 extend substantially in the thickness direction TD rather than in parallel to the upper surface A and lower surface B as viewed in a cut surface taken in parallel to the cross direction CD of the nonwoven fabric 1. According to the present invention, the liquid-pervious ability is evaluated in the form of liquid-permeating rate and a predominant orientation of the composite fibers 2 in the thickness direction TD is evaluated in the form of the average fiber angle $\theta$.

The composite fibers 2 have a fineness of 1 to 17 dtex and a fiber length of 10 to 150 mm. The composite fibers 2 comprise a core component and a sheath component wherein the sheath component is made of a thermoplastic synthetic resin selected to have a fusion point lower than a fusion point of a thermoplastic synthetic resin forming the core component so that the composite fibers 2 may be fused together at intersection points of these composite fibers 2 as the thermoplastic synthetic resin form the sheath component is fused. The preferred composite fibers 2 each comprise the core component and the sheath component in a concentric relationship with each other and presents no spiral crimp even when it is heated. It should be understood, however, that the composite fibers each comprising the core component and the sheath component in a slightly eccentric relationship with each other and presenting a negligible latent crimps under heating may be also used as the composite fibers 2 of this invention. Description "the composite fibers presenting a negligible latent crimps" used herein means that, after a web piece of 250 x 250 mm having a basis weight of 200 g/m$^2$ has been heated at a temperature of 145°C for 5 min, this web piece exhibits a contraction percentage of 5% or less in the first direction MD. According to the present invention, the composite fibers exhibiting such behavior in the web piece is generically named as the composite fibers each having the core component and the sheath component substantially in the concentric relationship with each other. The term "number of crimps" used herein refers to the value measured using the method specified in Section 8. 12 of JIS (Japanese Industrial Standards) L 1015.

Such nonwoven fabric 1 is suitably useful not only as liquid-pervious sheets in body fluid absorbent articles such as disposable diapers, sanitary napkins, panty liners or tampons or but also as liquid-pervious sheets, for example, body waste disposal sheets for pet animals. The nonwoven fabric 1 is useful as wipes to clean the human body or equipment. It should be noted here that, when the nonwoven fabric 1 is used as the liquid-pervious sheet for the purpose of covering the liquid-absorbent core in the bodily fluid absorbent article, the composite fibers 2 each preferably has a fineness of 2.6 to 4.4 dtex and a fiber length of 38 to 51 mm to achieve a soft touch of the nonwoven fabric 1. Furthermore, for this article, the composite fibers 2 are preferably coated with appropriate surfactant or plasma-treated in order to modify the fiber surface to be hydrophilic. If it is desired to improve a water-absorbing property of the nonwoven fabric 1, hydrophilic natural fibers such as cotton or hydrophilic semi-synthetic fibers such as rayon fiber up to 10% by weight may be added as the blending fibers to the composite fiber 2. While the thermoplastic synthetic resin constituting the core component and the sheath component of the composite fibers 2 may be selected from the group including olefin resins such as polyethylene or polypropylene, polyamide resins such as nylon, polyester resins and polyacrylonitrile resins, it is preferred to use polyethylene as the sheath component to assure that the composite fibers 2 are firmly fused together at the intersection points thereof at a relatively low temperature. For the nonwoven fabric 1 in which the composite fibers 2 are fused together by means of fused polyethylene at the intersection points thereof, it is required that the core component should not be fused even when the sheath component is fused. To meet this requirement, the core component is preferably formed by polypropylene or polyester having a fusion point substantially higher than a fusion point of the sheath component. The thermoplastic synthetic resin used to form the core component and/or the sheath component may contain particles of inorganic substance, for example, titanium oxide as filler. The filler has a particle size preferably in a range of 0.05 to 0.5 $\mu$m and such filler makes it possible to regulate respective degrees of glaze and transparency of the composite fiber 2. The nonwoven fabric 1 formed by the composite fibers 2 containing the filler may be advantageously used as liquid-pervious sheets wrapping liquid-absorbent cores to hide any contamination of the core due to bodily fluids.

Fig. 2 shows a production process to obtain the nonwoven fabric 1 using the composite fibers 2, including the steps of obtain the composite fibers 2. In a step I of the production process shown in Fig. 2, the high fusion point resin as material for the core component and the low fusion point resin as the material for the sheath component are subjected to melt extrusion followed by melt spinning to obtain filamentous composite fibers 2a from which the composite fibers 2 will be obtained. The high fusion point resin and/or the low fusion point resin in this step I may contain the inorganic filler such as titanium oxide at a rate of content so limited not to interfere with the melt spinning of the composite fibers 2a and a stretching treatment of the composite fibers 2a in a subsequent step III.

In a step II, the composite fiber 2a is paralleled to obtain a tow 2b.

In the step III, a tow 2b is heated at a desired temperature and subjected to a primary stretching treatment and a secondary stretching treatment in order to regulate fineness and strength of the composite fibers 2a.

In a step IV, the tow 2b coated with lubricant is fed into a box-type crimper and therein mechanically crimped.

In a step V, the tow 2b is subjected to an annealing treatment. Specifically, the tow 2b is heated in its relaxed condition so that the crimps are fixed and simultaneously the tow 2b is heat-contracted to stabilize a configuration of the tow 2b.

In a step VI, the tow 2b is cut into a desired length and an assembly of the composite fibers 2 in the form of staples.

In a step VII, an assembly of the composite fiber 2 is guided through a card 101 to fibrillate the assembly and thereby to obtain web 102 comprising the composite fibers 2.

In a step VIII, the web 102 is placed on an endless belt 103 serving as a support used to convey the web 102 in the machine direction MD.

In a step IX, the web 102 is exposed to a blast of pressurized air blown downward with a pretreatment chamber 104 to move the composite fibers 2 constituting the web 102 downward in the thickness direction TD of the web 102 to increase a density of the web 102. A temperature of the pressurized air should be limited not to fuse the sheath component.

Within the pretreatment chamber 104, the pressurized air is suctioned from below the web 102.

In a step X, the web 102 is exposed within treatment chambers 105, 106, 107 following the pretreatment chamber 104 to a blast of heated air from above to fuse the low fusion point resin forming the sheath component of the composite fiber 2 and the fused resin is utilized to fuse the composite fibers 2 together at the intersection points thereof. Within the respective treatment chambers 105, 106, 107, the heated air is suctioned from below the web 102. While a temperature as well as a flow rate of the heated air within the respective treatment chambers 105, 106, 107 may be selectively adjusted, the temperature of the heated air must be at a fusion temperature of the low fusion point resin or higher.

In a step XI, the web 102 exiting the treatment chamber 107 is cooled to obtain the nonwoven fabric 1 and this nonwoven fabric 1 is reeled.

Referring to Fig. 2, a stretching treatment of the tow 2b in the step III may be achieved by the primary stretching treatment alone and simplified by saving a secondary stretching treatment. For the nonwoven fabric 1 comprising, in addition to the composite fiber 2, thermoplastic synthetic fibers or hydrophilic fibers as blending fibers 112, such blending fibers 112 also may be guided through the card together with an assembly of the composite fibers 2 in the step VII to fibrillate the blending fibers 112 also. To facilitate the blending fibers to be guided through the card and to be blended with the composite fibers 2, the blending fibers 112 has preferably been mechanically crimped prior to this step VII. The step IX is an optional step and may be saved when it is unnecessary to increase a density of the web 102 prior to the step IX. In the step IX, the pressurized air may be replaced by mechanical means such as an embossing roll to compress the web 102 in the thickness direction and thereby to increase a density thereof. It is also possible to use a heated embossing roll or an embossing roll of supersonic vibration type so that the web 102 may be partially compressed and the composite fibers 2 may be fused together in the respective compressed regions of the web 102.

A specific embodiment of the production process of Fig. 2 using the composite fibers 2 consisting of polyester as the core component and polyethylene as the sheath component proceeds as will be described hereunder.

In the step I, the composite fibers 2a comprising core-sheath type filaments wherein the core component and the sheath component are substantially in concentric relationship with each other are melt spun, using polyester as the high fusion point resin and polyethylene as the low fusion point resin. While the polyethylene may be selected from the group consisting of high density polyethylene, low density polyethylene, straight-chain low density polyethylene and mixture thereof, it is preferred to use high density polyethylene having a density of 0.95 to 0.97 $g/cm^3$ and a melt flow rate specified by JIS K 7210 in a range of 10 to 30 g/10 min.

In the step II, the composite fibers 2a are paralleled to obtain the tow 2b.

In the step III, the tow 2b is stretched by 130 to 400% at a temperature of 70 to 110°C so that the composite fibers 2a constituting the tow 2b may take the form of filament having a fineness in a range of 1 to 17 dtex, more preferably in a range of 2 to 10 dtex.

In the step IV, the stretched tow 2b is overfed into the box-type crimper and thereby the composite fibers 2a is mechanically crimped so as to repeat curvature in zigzags at a rate of 10 to 35 curvatures/25 mm, more preferably at a rate of 13 to 20 curvatures/25 mm.

In the step V, the crimped tow 2b is annealed by heating the tow 2b at a temperature of 120°C for 5 to 8 minutes.

In the step VI, the annealed tow 2b is now cut into an absolute size of 10 to 150 mm, more preferably of 25 to 65 mm and thereby to obtain the assembly of the composite fibers 2 in the form of staples.

In the step VII, the assembly of the composite fibers 2 is fibrillated to obtain the web 102. As will be appreciated by those skilled in the art, it is also possible to obtain the web 102 from the composite fibers 2 containing therein the blending fibers 112. As the blending fibers 112, thermoplastic synthetic fibers such as composite fibers or hydrophilic natural fibers such as cotton or hydrophilic semi-synthetic fibers such as rayon, in any case, having a fiber length of 10 to 55 mm and latent crimps may be used. In the nonwoven fabric 1, a content of the blending fibers 112 is preferably 50% or less by weight if the thermoplastic synthetic fibers are used and preferably 10% or less by weight if the natural fiber or the semi-synthetic fibers are used with respect to a total weight of the nonwoven fabric 1. The term "the composite fibers used as the blending fibers 112 and having latent crimps" as used herein refers to the composite fibers reliably expressing the latent crimps under heating in the step X.

In the step IX, the web 102 is exposed to blast of hot air at a temperature limited not to fuse the sheath component of the composite fibers 2, for example, at a temperature in a range of 80 to 125°C, more preferably in a range of 90 to 110°C at a rate of 1.5 to 3 m/sec if the sheath component is of polyethylene and thereby the composite fibers 2 are moved downward without changing the mechanically crimped state in the thickness direction TD of the web 102.

In the step IX, the sheath components of the composite fibers 2 are fused together at the respective intersection points and thereby interlaced together. For example, if the sheath component is of polyethylene, the web 102 may be exposed to blast of hot air at a temperature of 130 to 150°C at a rate of 0.5 to 1.5 m/sec. For the web 102 containing therein the composite fibers having latent crimps as the blending fibers 112, such blending fibers 112 are fused with or mechanically interlaced with the composite fiber 2 under blast of hot air and simultaneously spirally crimped. Consequentially, the blending fibers 112 serve not only to restrain undesired movement of the composite fibers 2 but also to prevent orientation of the composite fibers 2 in the machine direction as well as in the cross direction from being changed in the step X.

In this production process, a mechanically crimped tow 2b is annealed under heating at a temperature corresponding to the fusion point of the sheath component constituting the composite fibers 2 or a temperature near to this fusion point. As a result, the crimped state of the composite fibers 2 in the form of staple can be thermally stabilized. In the course of running through the card 101 in the machine direction MD, the composite fibers 2 develops a strong tendency to extend in parallel to the machine direction MD and at the same time develops a strong tendency to repeat zigzag curvatures in the thickness direction TD. In the nonwoven fabric 1 obtained after leaving the step X, the crimps are apt to moderate under the effect of hot blast within the respective treatment chambers 105, 106, 107, but most of the composite fibers can maintain the tendency to extend in the machine direction MD with the repeated curvatures in the thickness direction TD.

With the composite fibers 2 thermally stabilized to be crimped in this manner, the tow 2b behaves to have decreased "deformation residual ratio" representing an elastic recovery. The nonwoven fabric 1 obtained such tow 2b behaves to have a large "specific volume" representing a ratio between volumes of the nonwoven fabric before and after placed under a load. In other words, the nonwoven fabric behaves to maintain a desired bulkiness even after placed under a load. Furthermore, when the nonwoven fabric 1 is placed on a horizontal plane and observed in a cut surface taken in parallel to the cross direction CD, "the fiber angle" defined by an angle at which the vertical line with respect to the horizontal plane intersects the composite fibers 2 have a tendency to narrow. Such tendency suggests the composite fibers 2 behaves to extend vertically as viewed in a cut surface of the nonwoven fabric 1 taken in parallel to the cross direction CD. In addition, when used as the liquid-pervious topsheet in a sanitary napkin, the nonwoven fabric 1 behaves to reduce "liquid permeation rate" for a specified quantity of artificial menstrual blood. Methods for measurement of these "deformation residual ratio", "specific volume", "average fiber angle" and "liquid permeation rate" will be described in details with respect to specific embodiments described hereunder.

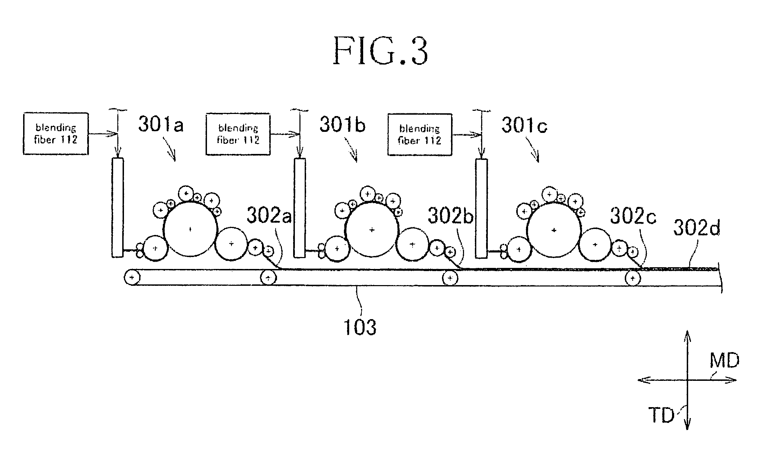

Fig. 3 shows another embodiment of a production process for making the nonwoven fabric 1 according to the present invention similar to the production process shown in Fig. 2 except that the card 101 in Fig. 2 is replaced by first, second and third cards 301a, 301b, 301c. Upstream of these first, second and third cards 301a, 301b, 301c, respectively, there are provided steps (not shown) corresponding to the step I through the step VI in Fig. 2 and the assemblies of the composite fibers 2 are fed to these first, second and third cards 301a, 301b, 301c. In this way, first web 302a, second web 302b and third web 302c comprising the composite fibers 2 are obtained. These first web 302a, second web 302b and third web 302c are placed one upon another on the endless belt running in the machine direction MD to form a laminate web 302d to be fed to steps corresponding to the step VIII and the subsequent steps in Fig. 2.

The production process shown in Fig. 3 may be useful when a processing capacity of the card is low and it is difficult for a single card to produce the desired web which is uniform in composition and relatively high in basis weight. For example, a first web 302a, a second web 302b and a third web 302c each having a basis weight of 10 $g/m^2$ may be obtained by the first, second and third cards 301a, 301b, 301c and then placed one upon another to obtain laminate web 302d and thereby the nonwoven fabric 1. In the production process of Fig. 3, the first web 302a, the second web 302b and the third web 302c may be provided with fiber composition differing one from another to ensure that the nonwoven fabric 1 has a variation in the fiber composition appearing in the thickness direction thereof. It should be understood here that the present invention is not limited to the embodiments shown in Figs. 2 and 3 and, for example, the number of the cards used in the process may be appropriately selected as circumstances demand.

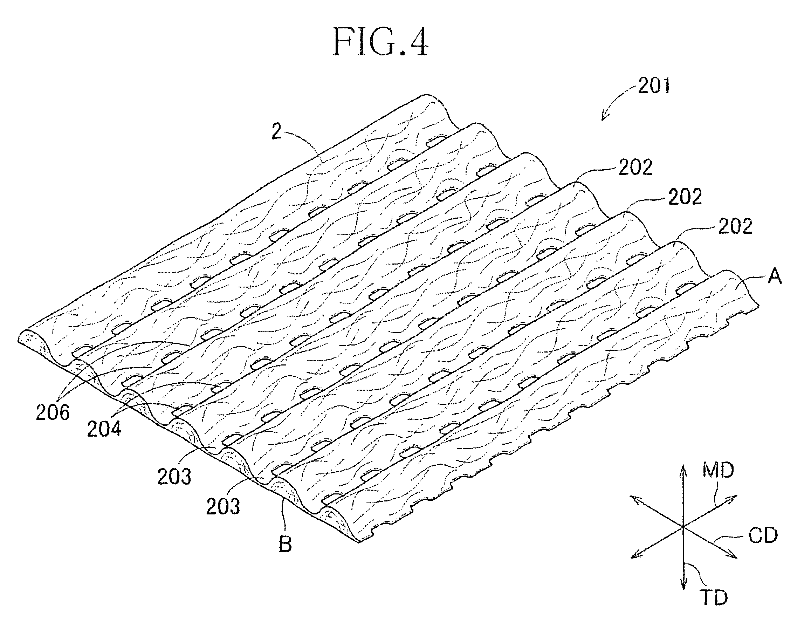

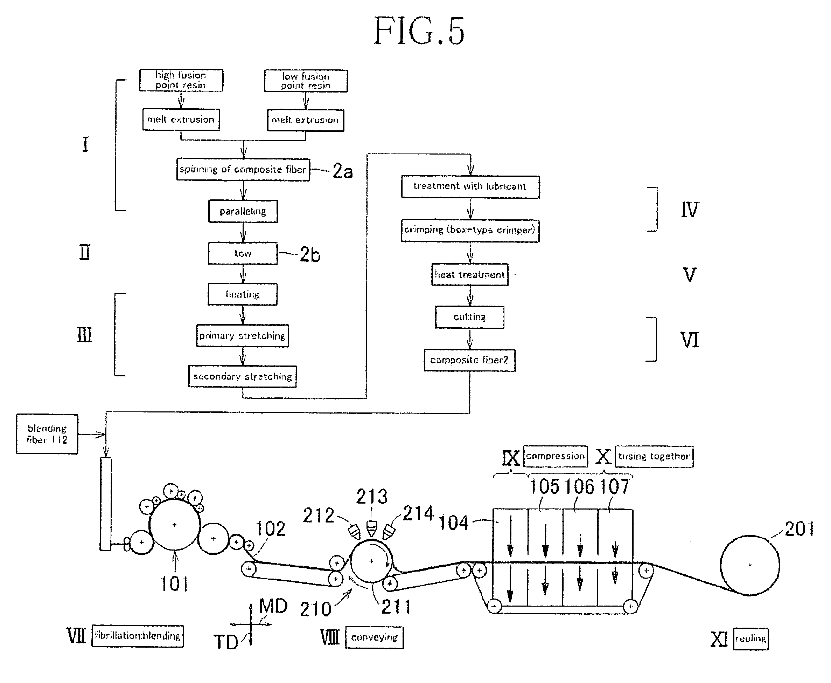

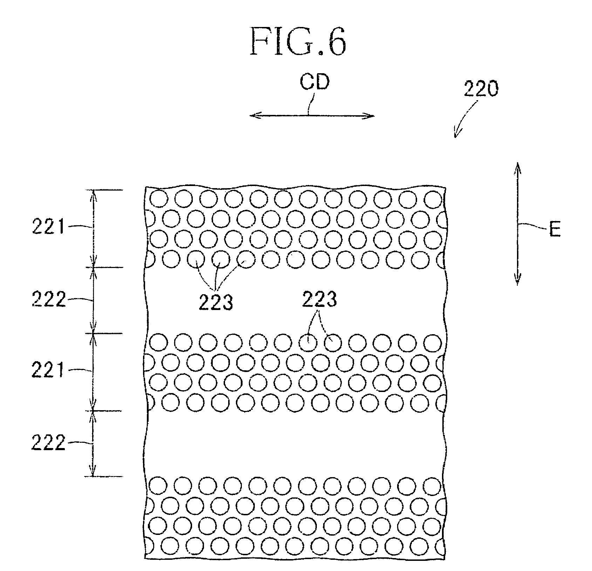

Fig. 4 is a perspective view showing a nonwoven fabric 201 according to one embodiment of the present invention, Fig. 5 is a schematic diagram showing a production process for this nonwoven fabric 201 and Fig. 6 is a schematic diagram partially showing various means used in this production process.

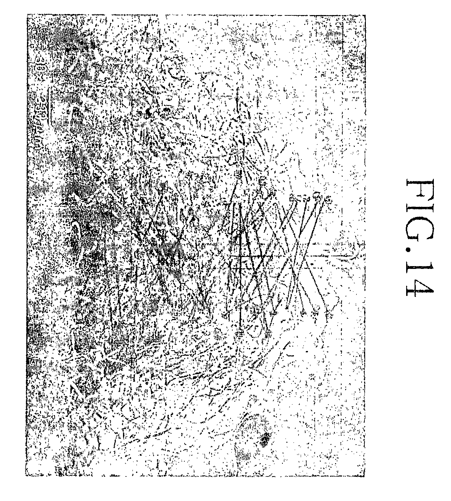

The nonwoven fabric 201 shown in Fig. 4 has been obtained using the same composite fiber as that shown in Fig. 14. The nonwoven fabric 201 is formed on its upper surface A with a plurality of crests 202 extending in parallel one to another in the machine direction MD and a plurality of troughs 203 extending also in parallel one to another in the machine direction MD so that these crests 202 and troughs 203 undulating in the cross direction CD. A lower surface B of the nonwoven fabric 201 is flat and, in the respective troughs 203, through-holes 204 extending through the upper and lower surfaces A, B are intermittently arranged in the machine direction MD.

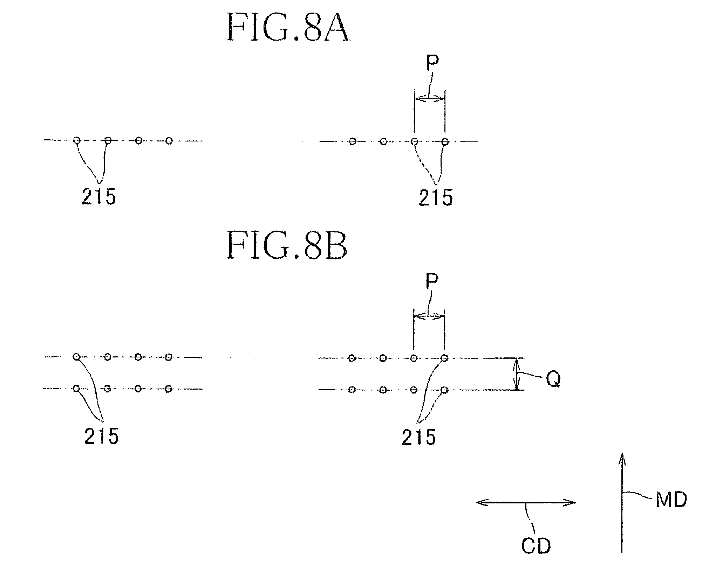

A production process of Fig. 5 is similar to that of Fig. 2 except that molding means 210 is additionally used in the step VIII between the step VII and the step IX. The molding means 210 used to form the nonwoven fabric 201 with the crests 202, the troughs 203 and the through-holes 204 comprises a suction drum 211 adapted to rotate in the machine direction MD and nozzle assemblies 212, 213, 214 adapted to blow out air blast. The nozzle assemblies 212, 213, 214 are respectively adapted to blow out air blast against a peripheral surface of the suction drum 211 and arranged at regular intervals in a circumferential direction of the suction drum 211 at a predetermined distance from the peripheral surface of the suction drum 211. Each of the nozzle assemblies 212, 213, 214 comprises, in turn, a plurality of individual nozzles 215 (See Fig. 8) arranged at regular intervals along an air supply piping (not shown) preferably in a manner that the individual nozzles 214 in each of the nozzle assemblies 212, 213, 214 lie in a straight line in the machine direction MD.

The nozzle assemblies 212, 213, 214 may be arranged to be spaced one from another, for example, by an angular distance of 30°C circumferentially of the suction drum 211 while the individual nozzles 215 in the respective nozzle assemblies 212, 213, 214 are arranged, for example, at a pitch of 5 mm in the cross direction along the air supply piping. The nozzle assemblies 212, 213, 214 are adapted to provide air blast heated at a desired temperature at a desired airflow rate. More specifically, blasts of hot air from the individual nozzles 215 are regulated so that these blasts of hot air themselves or interference among them may not disturb a distribution of the composite fibers 2 in the web 102. Assumed that the web 102 having a basis weight of 35 g/m$^2$ moves past the suction drum 211 having a diameter of 500 mm along a predetermined part of its peripheral surface in 0.5 sec and the individual nozzles 215 in the respective nozzle assemblies 212, 213, 214 are arranged at a pitch of 5 mm in the cross direction CD at a distance of 5 to 8 mm from the peripheral surface of the suction drum 211, the web 102 runs immediately under the individual nozzles 215 preferably after the web 102 has been adjusted under the suction effect to have a thickness of 5 to 8 mm. Preferably, in the course of such processing, the individual nozzles 215 has a diameter in the order of 0.5 to 1.5 mm, the airflow rate from the individual nozzle is 50 to 700 m/sec and a sucking force of the suction drum 211 is 2 to 7 m/sec.

The suction drum 211 in Fig. 5 is provided on its peripheral surface with a molding plate 220 as shown in Fig. 6. The plate 220 is formed with perforated zones 221 and imperforated zones 222 alternating in a circumferential direction E of the suction drum 211. Each of the perforated zones 221 includes a plurality of through-holes 223 fluid-communicated with a suction mechanism (not shown) of the suction drum 211. In a specific embodiment of the plate 220, each of the respective perforated zones 221 has a dimension of 2 to 3 mm in the circumferential direction E and extends substantially over an entire dimension of the suction drum 211 in an axial direction, i.e., the cross direction of the suction drum 211. In each of the perforated zones 221, a plurality of the through-holes 223 each having a diameter of 0.2 to 1 mm are formed at an opening ratio of 15 to 30% with respect to a total area of the perforated zone 221. Each of the imperforated zones 222, on the other hand, has a dimension of 1.5 to 3 mm in the circumferential direction E and fully extends in the axial direction of the suction drum 211. The suction drum 211 having the plate 220 mounted thereon has a circumferential velocity corresponding to a velocity at which the web 102 is conveyed.

In the production process of Fig. 5, the web 102 having a uniform thickness is formed via the steps I through VII corresponding to those in the production process of Fig. 2. In the step VIII, the web 102 runs past the molding means 210 wherein the web 102 is placed on and conveyed by the peripheral surface of the suction drum 211 so as to run immediately under the nozzle assemblies 212, 213, 214. Simultaneously, the web 102 is exposed to air blasts blown out from the respective nozzle assemblies 212, 213, 214 while this air is sucked by the suction drum 211.

In the web 102 exposed to the air blast, the composite fibers 2 just underlying the individual nozzles 215 are moved in parallel one to another in the cross direction CD and gathered together between respective pairs of the adjacent individual nozzles 215, 215 to form a crest (not shown) corresponding to the crest 202 as seen in Fig. 4, on one hand, and to form, immediately under the individual nozzles 125, troughs (not shown) corresponding to the troughs 203 as seen in Fig. 3, on the other hand. However, in the imperforated zones 222 of the molding plate 220 defining the peripheral surface of the suction drum 211, the air blasts blown out against the web 102 are guided not into the suction drum 211 but along the surface of the molding plate 220 in the cross direction CD. The web 102 is formed with through-holes (not shown) corresponding to the through-holes 204 of Fig. 4 as almost every one of the composite fibers 2 overlying the imperforated zones 222 is moved by the air in the cross direction CD. The moiety of the composite fibers extending in the perforated zones 221 partially remain immediately under the individual nozzles 215 without moving in the cross direction as most of the air blasts blown out from the individual nozzles 215 against these composite fibers flows into the suction drum 211 via the through-holes 223. As a result, bridges (not shown) corresponding to bridges 206 (See Fig. 4) connecting each pair of the adjacent crests 202 with each other are formed.

As will be apparent from Fig. 4, the trough 203 includes the through-holes 204 and the bridges 206 formed in the manner as has been described just above. The temperature of the air blasts from the nozzle assemblies 212, 213, 214 is adjusted preferably in a range of 90 to 250°C in the case of the composite fiber 2 consisting of polyester as the core component and polyethylene as the sheath component. Generally, the composite fibers 2 may be exposed to the air blasts at a sufficiently high temperature to fuse the sheath components of the composite fibers to ensure that, in the web 102 having the surface morphology as shown in Fig. 4 given by the molding means 210 in the previous step, the sheath components of the composite fibers 2 lying immediately under the individual nozzles 215 are fused together. Consequentially, not only the surface morphology can be reliably maintained in the step VIII and the subsequent steps but also a degree of compression of the web 102 along the respective crests can be increased in the step IX. According to the present invention, such treatment of heating the web 102 performed in the step VIII prior to the step IX is referred to as "preheating".

It is possible to increment the temperature of the air blasts blown out from the nozzle assemblies 212, 213, 214 in this order. In this case, the moiety of the composite fibers 2 underlying the individual nozzles 215 may be moved in the cross direction under the effect of the air blasts from the nozzle assemblies 212, 213 located on the side of an entranceway of the web 102 and gathered together between each pair of the individual nozzles 215 adjacent to each other in the cross direction. Temperature and airflow rate of the air blasts from the nozzle assemblies 212, 213 may be regulated so that the web 102 is heated but the sheath component of the composite fibers 2 might not be fused. For the composite fibers 2 each consisting of polyester as the core component and polyethylene as the sheath component, the air blasts may be adjusted to have a temperature in a range of 90 to 200°C. The air blast from the nozzle assembly 214 primarily serves to fuse the sheath components underlying the individual nozzles 215 together and thereby to stabilize a configuration of the web 102. Temperature of the air blasts for this purpose is higher than the temperature of the air blasts from the nozzle assemblies 212, 213 and may be adjusted to a range of 180 to 250°C.

In regard to the nozzle assemblies 212, 213, 214, an alternative embodiment is possible without departing from the scope of the invention. According to this alternative embodiment, the diameter of the individual nozzle 215 in the respective nozzle assemblies may be gradually enlarged in the order of the assemblies 212, 213, 214 and thereby an area of the web 102 exposed to the air blast from the individual nozzle 215 may be gradually enlarged in the cross direction CD. In this way, the width of the trough formed in the web 102 can be gradually enlarged in the cross direction CD in the step of obtaining the nonwoven fabric 201 of Fig. 4. For example, the individual nozzle 215 having a diameter of 0.7 mm may be used in the nozzle assembly 212 and the individual nozzle 215 having a diameter of 1.0 mm may be used in the nozzle assemblies 213, 214. The distribution of the composite fibers should not be significantly disturbed in the machine direction MD, the cross direction CD and the thickness direction TD as the composite fibers 2 of the web 102 move past the steps IX and X so far as the web 102 is processed in the manner as has been described just above using the molding means 210.

The step of forming the web 102 with the crests and the troughs both extending in the machine direction MD using the nozzle assemblies 212, 213, 214 is applicable also to the case in which the web 102 contains the latently crimped fibers as the blending fibers 112. In the web 102 obtained by blending the composite fibers 2 with the blending fibers 112 in the step VII of Figs. 2 and 5, there is a possibility that the blending fibers 112 are not uniformly distributed but locally concentrated. Assumed that such web 102 is heated and, as a result, the latently crimped blending fibers 112 are spirally crimped, these blending fibers 112 now having apparent length thereof reduced will function to pull the composite fibers 2 in various directions, leading to a significant changed distribution pattern of the composite fibers 2 in the web 102 immediately after the web 102 has moved past the card 101.

Such problem is avoided, for example, by previously forming the web 102 with the crests and the troughs using the nozzle assemblies 212 and 213 so as to gather the composite fibers 2 together with the composite fibers 112 in the crests, then preheating the web 102 using the nozzle assembly 214 and thereby fusing the fibers in the web 102 together at a slight degree. In such production process, most of the blending fibers 112 are spirally crimped and the apparent dimension of the blending fibers 112 is reduced along the relatively narrow crests of the web 102 under the effect of this preheating. In such production process also, the phenomenon that the composite fibers 2 are pulled by the blending fibers 112 in the various directions of reduction occurring in the apparent dimension of the blending fibers 112. However, this phenomenon occurs not evenly over a large extent of the web 102 but only in the crests. By forming the web 102 with the crests using the latently crimped blending fibers 112 in this manner, the distribution of the composite fibers 2 in the nonwoven fabric 201 obtained from the web 102 can be concentrated in the crests 202. When the latently crimped composite fibers are used as the blending fibers 112, the blending fibers 112 are crimped and thereby dimension-reduced often at a very irregular contraction percentage. However, in the case of the web 102 formed with the crests as the web 102 moves past the molding means 210, most of the blending fibers 112 is concentrated in the crests and therefore a contraction percentage of the blending fibers 112 presents an averaged value. So far as the nonwoven fabric 201 obtained from such web 102 is concerned. The nonwoven fabric 210 should not be remarkably affected by a portion of the blending fibers 112 having a significantly high contraction percentage.

The latently crimped fiber used as the blending fibers 112 may be selected from the group including eccentric core-sheath type composite fibers, eccentric core-sheath type hollow composite fibers and side-by-side type composite fibers wherein the latently crimped composite fibers to be selected has preferably a web contraction percentage (described later in detail) in a range of 10 to 40%. If the web contraction percentage of the latently crimped composite fibers is less than 10%, a contraction percentage of the apparent dimension thereof as the crimps are actualized will be too low to gather the composite fibers 2 together and correspondingly to assist the composite fibers 2 to be interlaced one with another. If the web contraction percentage exceeds 40%, on the contrary, a diameter of each spiral of the latently crimped composite fibers as the crimps are actualized will be often reduced and apt to lay down, in other words, the average fiber angle $\theta$ will be disadvantageously enlarged. In the latently crimped composite fibers suitably useful as the blending composite fibers 112, a volume ratio between the core component and the sheath component is preferably adjusted in a range of 50:50 to 70:30 so that a sufficient volume of the sheath component can be assured to facilitate such latently crimped composite fibers to be fused together with the composite fibers 2. Furthermore, the latently crimped composite fibers preferably has a fiber length in a range of 38 to 64 mm and fineness in a range of 1.5 to 4 dtex to increase the number of points at which the latently crimped composite fiber is fused together with the composite fiber 2.

Referring to Fig. 5, while the web 102 is processed to the nonwoven fabric 201 via the steps IX, X, XI similar to those illustrated by Fig. 2, the plate 220 of Fig. 6 may be replaced by a plate formed over its whole area with the through-holes 223 and having none of the imperforated zones 222. Use of such plate 220 leads to the nonwoven fabric 201 formed with the crests 202 and the troughs 203 but not with the through-holes 204.

In the crests 202 of the nonwoven fabric 201 obtained in this manner, the composite fibers 2 extend in the thickness direction TD in the same pattern as in the nonwoven fabric 1 of Fig. 1. Specifically, the nonwoven fabric 201 presents a relatively large "specific volume", a relatively small "average fiber angle" in the crests 202 and a relatively low "liquid-permeation rate". Of the composite fibers 2 extending in the crests 202, those lying in the vicinity of the troughs 203 are considered to be moved in the cross direction CD under the effect of the air blasts in the molding means 210. Observation of the crest 202 appearing in the cut surface of the nonwoven fabric 201 taken in parallel to the cross direction CD indicates that the composite fibers 2 are apt to be moved in the thickness direction TD under the air blasts.

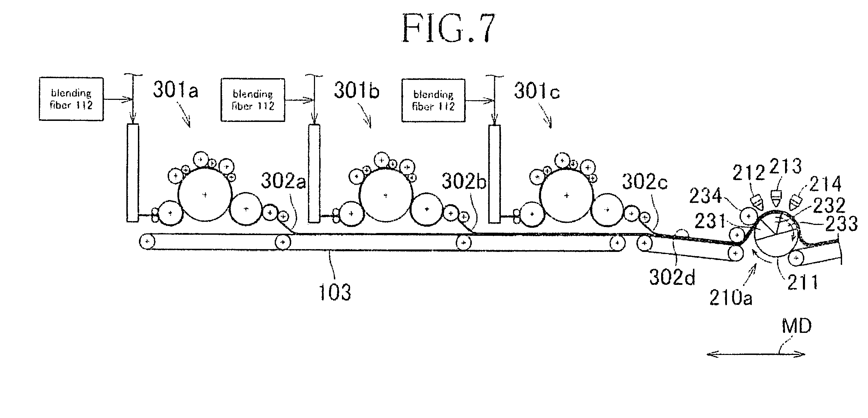

Fig. 7 partially illustrates one embodiment of the production process similar to the production process of Fig. 5 for the nonwoven fabric 201 according to the invention except that the card 101 of Fig. 5 is replaced by the first, second and third cards 301a, 301b, 301c exemplarily shown in Fig. 3.

Furthermore, in the production process of Fig. 7, the molding means 201 shown in Fig. 5 is replaced by molding means 210a. The molding means 210a has a deaerating roll 234 upstream of the nozzle assembly 212 while the suction drum 211 comprises a first suction zone 231, a second suction zone 232 and a third suction zone 233. These first, second and third suction zones 231, 232, 233 have individually controllable sucking force wherein the first suction zone 231 is opposed to the deaerating roll 234, the second suction zone 232 is opposed to the nozzle assemblies 212, 213 and the third suction zone 233 is opposed to the nozzle assembly 214. The deaerating roll 234 is formed on its peripheral surface with a plurality of through-holes (not shown) having a diameter, for example, of 5 mm at an area ratio of 30% and rotating at a circumferential velocity corresponding to 105 to 120% of a traveling speed of the endless belt 103 so that the laminate web 302d may be kept in contact with the peripheral surface of the suction drum 211 in a state of tension in the machine direction MD.

Referring to Fig. 7, assumed that the laminate web 302d having a basis weight of 35 g/m$^2$ travels past the peripheral surface of the suction drum 211 having a diameter of 500 mm in 0.5 sec, the deaerating roll 234 having a diameter of 200 mm is preferably operated at a distance of approximately 3 mm from the peripheral surface of the suction drum 211. Use of such deaerating roll 234 facilitates it to decrease a thickness of 30 to 40 mm of as measured immediately before the molding means 210 to a thickness of 2 to 5 mm. Referring also to Fig. 7, the first, second and third suction zones 231, 232, 233 of the suction drum 211 are preferably adjusted so that these suction zones 231, 232, 233 may have sucking forces of 5 to 10 m/sec, 2 to 5 m/sec and 5 to 7 m/sec. Even in such case, the sucking forces of the first and third suction zones 231, 233 may be set to relatively high levels while the sucking force of the second suction zone 232 may be set to a relatively low level to obtain a desired effect. More specifically, with the laminate web 302d kept in close contact with the peripheral surface of the suction drum 211 upstream and downstream of the second suction zone 232, the composite fibers 2 and the blending fibers 112 in the laminate web 302d are moved in the cross direction CD under the operation of the nozzle assemblies 212, 213 so that the crests 202 and the troughs 203 in the nonwoven fabric 201 may be easily formed.

It is also possible to obtain the nonwoven fabric 201 by using the first, second and third cards 301a, 301b, 301c in a manner as follows. The first card 301a supplies the first web 302a comprising the composite fibers each having a relatively short fiber length, for example, in a range of 15 to 44 mm, a small number of mechanical crimps in a range of 10 to 15/25.4 mm and a basis weight of 10 g/m$^2$. The second card 301b supplies the second web 302b comprising the composite fibers having a relative long fiber length, for example, in a range of 44 to 64 mm, a large number of mechanical crimps in a range of 15 to 35/25.4 mm and a basis weight of 10 g/m$^2$. The third card 302c supplies the third web 302c identical to the second web 302b. The laminate web 302d comprising these first, second and third web 302a, 302b, 302c are exposed to the air blasts from the nozzle assemblies 212, 213, 214 and to suction effect by the suction drum 211 while the laminate web 302d is moved on the peripheral surface of the suction drum 211. In the course of such processing, the composite fibers 2 each having a relatively short fiber length and forming the first web 302a has a tendency to extend in the machine direction MD and simultaneously has a remarkable tendency to express the mechanical crimps in zigzags in the plane extending orthogonally to the horizontal endless belt 103. These tendencies are effective to improve a bulk retention ratio and to decrease the average fiber angle θ of the nonwoven fabric 201 so as to improve the liquid-permeation rate. On the other hand, the composite fibers 2 each having a relative long fiber length and forming the third web 302 effectively serves to prevent the composite fibers 2 from fluffing on the surface of the crest, to increase a fiber density on the surface of the crest and to improve appearance of the nonwoven fabric 2.

Fig. 8A and 8B are partial diagrams exemplarily showing two considerable arrangements of the individual nozzles 215 employed in the nozzle assemblies 212, 213, 214 shown in Figs. 5 and 7. In Fig. 8A, the individual nozzles 215 arranged on a line in the cross direction CD, for example, the individual nozzles 215 each having a diameter of 1 mm are arranged at a pitch P of 5 mm. In Fig. 8B, the individual nozzles 215 are arranged to form a double line so that each of the individual nozzles 215 arranged in the first line and each of the individual nozzles 215 arranged in the second line and being adjacent to the individual nozzle 215 in the first line are aligned with each other on a line extending in the machine direction MD. In Fig. 8B, the individual nozzles 215, for example, each having a diameter of 1 mm are arranged in the cross direction CD at a pitch P of 5 mm so that each pair of the adjacent individual nozzles 215 in the machine direction MD are spaced from each other at a center distance Q of 5 mm. The arrangement of the individual nozzles 215 in the nozzle assemblies 212, 213, 214 is not limited to these embodiments as illustrated by Fig. 8A and Fig. 8B and, for example, it is also possible to employ the arrangement of Fig. 8A for the nozzle assemblies 212, 213 and to employ the arrangement of Fig. 8B for the nozzle assembly 214 so that the composite fibers 2 and the blending fibers 112 left free in the troughs of the laminate web 302d formed by the nozzle assemblies 212, 213 may be assisted by the nozzle assembly 214 to be reliably fused together.

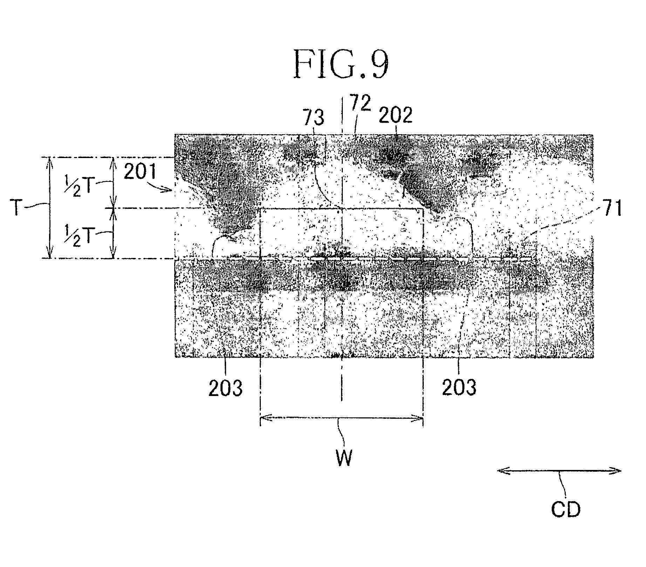

Fig. 9 is a sectional view (photo) taken in the cross direction CD of the nonwoven fabric 201 obtained using the production process shown in Fig. 7 wherein the nonwoven fabric 201 is placed on a horizontal plane 71 indicated by a chained line. As seen in this photo, the crests 202 and the troughs 203 appear alternately in the cross direction CD. The crest 202 has a width W as measured in the cross direction at a level defined by T/2 wherein T represents a height of the crest 202 from the horizontal plane 71 to an apex of the crest 202. The height T as well as the width of the crest 202 may be variably controlled depending on various parameters in the production process of Fig. 7 such as the pitch at which the individual nozzles 215 are arranged in the respective nozzle assemblies 212, 213, 214, the air blast rates from the individual nozzles 215, and a ratio feeding speed ratio of the web 102 between the steps X and XI. The inventors have found that a value of the average fiber angle $\theta$ evidently depends on the value of T/W. When it is desired to improve a transparency of the nonwoven fabric 201, the average fiber angle $\theta$ is preferably limited to 75° or less. In one embodiment of the invention, the nonwoven fabric 201 having a such value of the average fiber angle $\theta$ can be obtained by selecting the value T/W in a range of 0.55 to 1.00.

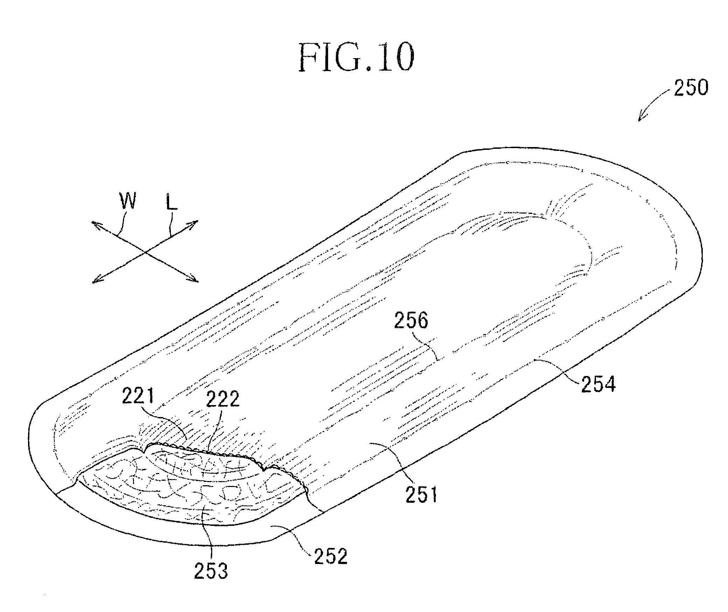

Fig. 10 is a partially cutaway perspective view of a sanitary napkin 250 as one example of the article using the nonwoven fabric 201. The sanitary napkin 250 comprises a liquid-pervious topsheet 251, a liquid-impervious backsheet 252 and a body fluid absorbent core 253 sandwiched between the topsheet 251 and the backsheet 252. The topsheet 251 and the backsheet 252 extend outward beyond a peripheral edge of the core 253, put flat together and bonded together at sealing spots 254 outside the peripheral edge of the core 253. In addition, the topsheet 251, the backsheet 252 and the core 253 assembled in this manner are heated and pressed together along a compressed line 256 describing an oval to ensure that this assembly is reliably integrated. As the topsheet 251, the nonwoven fabric 201 exemplarily shown in Fig. 4 is used. The crests 202 as well as the troughs 203 in the nonwoven fabric 201 extend in its longitudinal direction L of the sanitary napkin 250. As the backsheet 252, a plastic film is used and the core 253 is formed by a mixture of fluff pulp and super-absorbent polymer particles (both not shown) wrapped with tissue paper (not shown). In the topsheet 251, the composite fibers 2 of the nonwoven fabric 201 has repetitive curvatures in the thickness direction TD of the nonwoven fabric 201 so that most of menstrual blood may be rapidly absorbed by the core 253 through the topsheet 251 and scarcely spread in the longitudinal direction L as well as in the transverse direction W of the sanitary napkin 250. In the core 253, the menstrual blood quickly spreads over the tissue paper and then quickly absorbed and reliably retained by the mixture of pulp and super-absorbent polymer particles. Therefore, the menstrual bloodshould not stay after permeation through the topsheet 251 and eventually flow back through the topsheet 251 so as to let the wearer's skin wet. In this way, the sanitary napkin 250 using the nonwoven fabric 201 allows the menstrual blood to be absorbed in a strictly limited area of the topsheet 251. In other words, the sanitary napkin 250 presents a high performance of spot-absorption and a high performance of preventing the menstrual blood once absorbed from flowing back, i.e., rewetting phenomenon.

EMBODIMENTS

TABLES 1 and 2 list component fibers and performance evaluation results thereof in various embodiments of the nonwoven fabric obtained by the production process shown in Figs. 2, 3, 5 and 7 according to the present invention and the nonwoven fabric according to several comparative embodiments.

TABLES 1 and 2 contain therein entries as follow:

1. Composite fibers I and composite fibers II

These are used as the composite fibers 2 in the embodiments of the present invention.

2. Blending fiber

This is latently crimped fiber used as the blending fibers 112 in the embodiments of the present invention.

3. Fiber length

A length of fiber forced to be linear.

4. The number of crimps

The number of mechanical crimps of fibers included in a tow treated by the box-type crimper after spinning counted in accordance with method prescribed in JIS L 1015 with a pair of fiber-grips spaced from each other by a distance of 25 mm.

5. Heat treatment after crimping

A temperature at which the tow is heated in its relaxed state for 7 min for annealing treatment after having been taken out from the crimper.

6. Web's contraction percentage

From web comprising latently crimped fiber and having a basis weight of 200 g/m$^2$, a test sheet of 250 x 250 mm is prepared. A contraction percentage of this test sheet in the machine direction MD after this test sheet have been heat treated at a temperature of 145°C for 5 min.

7. Deformation residual ratio

(1) In the step V of Fig. 2, the heat treated tow of 120000 dtex is collected and vertically suspended. This tow is applied with a load of 24 g and provided at upper and lower points along this suspended tow with 100 mm indicator marks.
(2) The tow is additionally applied with a load of 75 g and heated at a temperature of 120°C for 5 min.
(3) After the tow has been cooled to a room temperature, the load of 75 g is removed, then a distance d (mm) between the upper and lower marks is measured and the deformation residual ratio (%) is calculated from a following equation:

$$(d - 100)/100 = \text{deformation residual ratio (\%)}$$

8. Fusion heat quantity

(1) Approximately 2 mg of composite fibers is sampled from the tow heat treated in the step V.
(2) Based on this sample, DSC (Differential Scanning Calorimeter) is used to measure a fusion heat quantity (J) and thereby to determine a first peak value in the course of a temperature rising process. This first peak value is divided by a weight (g) of the sample to obtain a fusion heat quantity $\Delta H$(J/g) of low fusion point resin constituting the composite fiber, for example, polyethylene.
(3) Measuring instrument and measuring condition:

Measuring instrument: Differential scanning calorimeter DSC-60 of Shimadzu Corporation
Sample container: Model PN/50-020 (container having a capacity of $15\mu\ell$) and Model PN/50-021 (crimp cover for the container)
Temperature rising rate: 5°C/min
Range of measured temperature: 50 to 200°C
Ambient atmosphere for measurement: nitrogen gas

9. Thickness of web after passing through the card

(1) The web having a basis weight of 30 g/m$^2$ leaving the card of the step VII is cut into 300 x 300 mm to prepare a sample.
(2) Thickness of the sample is measured under a load of a load of 0.1 g/cm$^2$ and a measured value is recorded as the thickness of the web after it has moved past the card.

10. Bulk retention ratio of web

(1) Seven(7) samples each prepared by cutting the web having a basis weight of 30 g/m$^2$ leaving the card of the step VII are placed one upon another and a thickness $h_0$ of this sample laminate is measured under a load of 0.1 g/cm$^2$.
(2) The sample laminate under the load is treated in a heating furnace at a temperature of 135°C for 5 min and then a thickness $h_1$ is measured after the sample laminate has been cooled.
(3) The bulk retention ratio is obtained from an equation of

$$\mathtt{h_1/h_0 \times 100 = bulk\ retention\ ratio\ (\%)}$$

11. Specific volume

(1) The nonwoven fabric is cut into 100 x 100 mm for sample preparation. Ten (10) samples are placed one upon another and a thickness of this sample laminate is measured under a load of 2000 gf. 1/10 of the measured thickness is recorded as the thickness t of the nonwoven fabric.
(2) A basis weight w of the nonwoven fabric is calculated from a weight of the 100 x 100 mm sample in units of g/m$^2$.
(3) t/w = specific volume (cm$^3$/g) is calculated as the specific volume under a load of 20 gf/cm$^2$.

12. Liquid-permeation rate

(1) The topsheet of a commercially available sanitary napkin (SOFY FUWAFUWA SURIMU, 25 cm manufactured by Uni-Charm Corporation) is replaced by a nonwoven fabric according to the embodiments of this invention or according to the comparative embodiments to prepare samples.
(2) An acrylic plate of 40 x 10 mm formed with a through-hole having a diameter substantially corresponding to a distal diameter of artificial menstrual blood dropping burette is placed upon the sample and a weight is placed on the acrylic plate so that the sample may be applied with a load of 2 gf/cm$^2$.
(3) Primarily, 3 m$\ell$ of artificial menstrual blood is dropped at a rate of 90 m$\ell$/min to the sanitary napkin via the through-hole and left for 1 min permeate the topsheet. The artificial menstrual blood used herein consists of 80 g of glycerin, 8 g of CMC sodium, 10 g of NaCl, 4 g of NaHCO$_3$, 8 g of red pigment No. 102, 2 g of red pigment No. 2 and 2 g of yellow pigment No. 5 mixed together and dissolved in 1000 cc of water.
(4) Secondarily, 4 m$\ell$ of artificial menstrual blood is dropped.
(5) With respect to each of the primarily dropped artificial menstrual blood and the secondarily dropped artificial menstrual blood, a length of time from a moment at which the artificial menstrual blood begins to be dropped to a moment at which the artificial menstrual blood completely permeates the topsheet into the core is measured. Both the liquid-permeation rates for the primarily and secondarily dropped artificial menstrual blood serve as indices for the high or low liquid-permeation rate of the nonwoven fabric.

13. Average fiber angle $\theta$

(1) The nonwoven fabric used as a sample for measurement is heated at a temperature of 70°C for 30 min to remove any traces of folding in the course of handling and thereby to flatten the sample.
(2) Standard substitute edge HA-100B for KOKUYO's cutter knife HA-7NB (Trade Name) is used to cut the sample in the cross direction CD to prepare a cut surface for observation extending in parallel to the cross direction CD and the sample is placed on a horizontal plane.
(3) The cut surface is observed through an electronic microscope (REAL-SURFACE-VIEW microscope VE-7800 manufactured by Keyence Corporation) and a 30 times magnified photograph of the cut surface is taken so as to bring an extent defined between upper and lower surfaces of the sample into view.
(4) At an optional position on the cut surface taken in photo, a vertical line with respect to the horizontal plane is drawn and auxiliary vertical lines are drawn on both sides of the primary vertical line so as to be spaced from this primary vertical line by 100 μm.

(5) Positions at which a single fiber intersects these two auxiliary lines are marked.

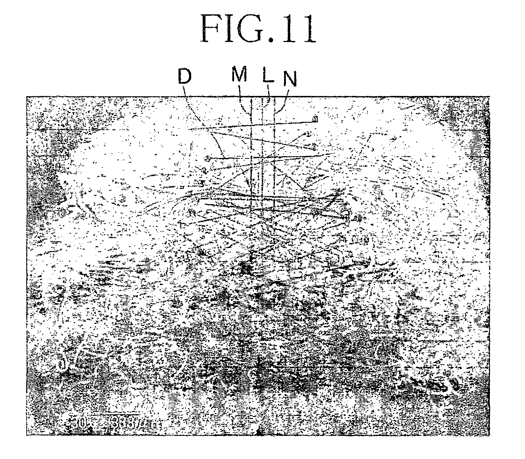

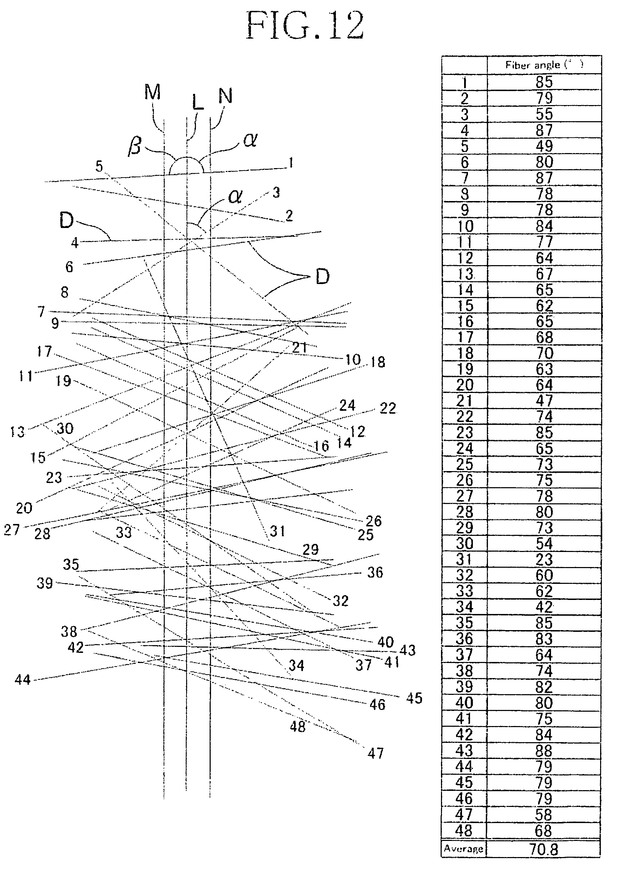

(6) The right and left marks are connected by a straight line and intersection angles α and β (See Figs. 11 and 12) between this straight line and the primary vertical line on both sides of the primary vertical line are measured. Of these intersection angles α and β measured in this manner, the angle of smaller value is determined as the fiber angle.

(7) Such fiber angle is obtained with respect to all the fibers sufficiently focused on the photo of the cut surface to be used as the obj ect to be measured and an arithmetic average value of the measured fiber angles is determined as "average fiber angle θ. These fibers well focused in the photo of the cut surface are referred to, according to the invention, as the fibers appearing in the cut surface.

## TABLE 1

| Component fibers | | | Unit | EX1 | EX2 | EX3 | EX4 | EX5 | EX6 | EX7 | EX8 | EX9 | EX10 | EX11 top | EX11 interm. | EX11 bottom | EX12 top | EX12 interm. | EX12 bottom | Comp. Ex.1 | Comp. Ex.2 | Comp. Ex.3 | Comp. Ex.4 |
|---|---|---|---|---|---|---|---|---|---|---|---|---|---|---|---|---|---|---|---|---|---|---|---|
| Fiber blending ratio | (composite fiber I):(composite fiber II):(blending fiber) | | weight ratio | 100:0:0 | 100:0:0 | 100:0:0 | 70:30:0 | 70:30:0 | 30:70:0 | 50:0:50 | 80:0:20 | 50:0:50 | 80:0:20 | 80:0:20 | 80:0:20 | 80:0:20 | 80:0:20 | 80:0:20 | 80:0:20 | 100:0:0 | 100:0:0 | 20:0:80 | 10:0:90 |
| Blending fiber | Heat treatment of tow after crimped | | °C | | | | | | | 90 | 90 | 90 | 90 | 90 | 90 | 90 | 90 | 90 | 90 | | | 90 | 90 |
| | Contraction rate | | % | | | | | | | 1 | 20 | 20 | 40 | 20 | 20 | 20 | 20 | 20 | 20 | | | 1 | 1 |
| | The number of mechanical crimps | | per 25mm | | | | | | | 15 | 15 | 15 | 15 | 15 | 15 | 15 | 15 | 15 | 15 | | | 15 | 15 |
| | Fiber length | | mm | | | | | | | 38 | 51 | 51 | 51 | 51 | 51 | 51 | 51 | 51 | 51 | | | 38 | 38 |
| | Finenesse | | dtex | | | | | | | 2.6 | 2.8 | 2.8 | 3.3 | 2.8 | 2.8 | 2.6 | 2.6 | 2.6 | 2.6 | | | 2.6 | 2.6 |
| | Sheath : core ratio | | volumeric ratio | | | | | | | 40:60 | 55:45 | 55:45 | 55:45 | 55:45 | 55:45 | 55:45 | 55:45 | 55:45 | 55:45 | | | 40:60 | 40:60 |
| | Core component | | | | | | | | | PET | PET | PET | PET | PET | PET | PET | PET | PET | PET | | | PET | PET |
| | Sheath component | | | | | | | | | PE | PE | PE | PE | PE | PE | PE | PE | PE | PE | | | PE | PE |
| Composite fiber II | Heat treatment of tow after crimped | | °C | | | | 120 | 120 | 120 | | | | | | | | | | | | | | |
| | Contraction rate | | % | | | | | | | | | | | | | | | | | | | | |
| | The number of mechanical crimps | | per 25mm | | | | 18 | 18 | 18 | | | | | | | | | | | | | | |
| | Fiber length | | mm | | | | 51 | 51 | 51 | | | | | | | | | | | | | | |
| | Finenesse | | dtex | | | | 2.6 | 2.6 | 2.6 | | | | | | | | | | | | | | |
| | Sheath : core ratio | | volumeric ratio | | | | 60:40 | 60:40 | 60:40 | | | | | | | | | | | | | | |
| | Core component | | | | | | PET | PET | PET | | | | | | | | | | | | | | |
| | Sheath component | | | | | | PE | PE | PE | | | | | | | | | | | | | | |
| Composite fiber I | Heat treatment of tow after crimped | | °C | 120 | 110 | 120 | 120 | 120 | 120 | 120 | 120 | 120 | 120 | 120 | 120 | 120 | 120 | 120 | 120 | 90 | 100 | 120 | 120 |
| | The number of crimps | | per 25mm | 15 | 15 | 15 | 15 | 15 | 15 | 15 | 15 | 15 | 15 | 18 | 18 | 15 | 18 | 18 | 15 | 15 | 15 | 15 | 15 |
| | Fiber length | | mm | 38 | 38 | 38 | 38 | 51 | 38 | 38 | 38 | 38 | 38 | 51 | 51 | 38 | 51 | 51 | 38 | 38 | 38 | 38 | 38 |
| | Finenesse | | dtex | 2.6 | 2.6 | 2.6 | 2.6 | 2.6 | 2.6 | 2.6 | 2.6 | 2.6 | 2.6 | 2.6 | 2.6 | 3.3 | 2.6 | 2.6 | 3.3 | 2.6 | 2.6 | 2.6 | 2.6 |
| | Sheath : core ratio | | volumeric ratio | 40:60 | 40:60 | 40:60 | 40:60 | 40:60 | 40:60 | 40:60 | 40:60 | 40:60 | 40:60 | 60:40 | 60:40 | 40:60 | 60:40 | 60:40 | 40:60 | 40:60 | 40:60 | 40:60 | 40:60 |
| | Core component | | | PET | PET | PET | PET | PET | PET | PET | PET | PET | PET | PET | PET | PET | PET | PET | PET | PET | PET | PET | PET |
| | Sheath component | | | PE | PE | PE | PE | PE | PE | PE | PE | PE | PE | PE | PE | PE | PE | PE | PE | PE | PE | PE | PE |
| Configuration of nonweven fabric | | | | FIG.1 | FIG.1 | FIG.4 | FIG.1 | FIG.1 | FIG.1 | FIG.1 | FIG.1 | FIG.1 | FIG.1 | FIG.4 | FIG.4 | FIG.4 | FIG.4 | FIG.4 | FIG.4 | FIG.1 | FIG.1 | FIG.1 | FIG.1 |
| Is stepIX included or not? | | | | yes | yes | no | no | no | no | no | no | no | no | yes | yes | yes | yes | yes | yes | no | no | no | no |
| Production process | | | | | | | | | | | | | | FIG.7 | FIG.7 | FIG.7 | FIG.7 | FIG.7 | FIG.7 | | | | |
| Basis weight of web | | | | 25 | 25 | 28 | 27 | 28 | 29 | 25 | 26 | 25 | 25 | 10 | 10 | 10 | 10 | 10 | 15 | 26 | 26 | 26 | 27 |

## TABLE 2

| | tow | | web | | nonwoven | | | | |
|---|---|---|---|---|---|---|---|---|---|
| | Deformation residual rate | Heat quantity of fusion for low fusion point resin — Heat quantity of fusion ΔH | Thickness after passage through card 30g/m² | Web's bulk retention rate 135°C 5min | Basis weight | Specific volume under a load of 20gf/cm² | Liquid-permeation rate Primary | Secondary | Average fiber angle θ |
| | % | J/g | mm | % | g/m² | ml/g | sec | sec | (°) |
| EXAMPLE1 | 18 | 176.4 | 26 | 45.0 | 25 | 41.3 | 11 | 17 | 70.8 |
| EXAMPLE2 | | 166.3 | | | 25 | 49.6 | 10 | 17 | 71.7 |
| EXAMPLE3 | | | | | 28 | 32.5 | 13 | 18 | 67.4 |
| EXAMPLE4 | | | | | 27 | 31.8 | 14 | 18 | |
| EXAMPLE5 | | | | | 28 | 30.9 | 11 | 18 | |
| EXAMPLE6 | | | | | 29 | | 10 | 18 | 72.0 |
| EXAMPLE7 | | | | | 25 | 37.4 | 9 | 17 | 74.7 |
| EXAMPLE8 | | | | | 26 | 40.7 | 9 | 17 | 69.3 |
| EXAMPLE9 | | | | | 25 | 39.1 | 9 | 16 | 71.6 |
| EXAMPLE10 | | | | | 25 | | | | |
| EXAMPLE11 (top layer / intermediate layer / bottom layer) | | | | | 30 | 42 | 8 | 17 | |
| EXAMPLE12 (top layer / intermediate layer / bottom layer) | | | | | 35 | 42 | 8 | 16 | |
| Comparative Ex.1 | 42 | 139.4 | 20 | 27.0 | 26 | 15.0 | 35 | 39 | 78.4 |
| Comparative Ex.2 | | 157.1 | | | 26 | | | | 79.4 |
| Comparative Ex.3 | | | | | 26 | | 16 | 18 | 77.5 |
| Comparative Ex.4 | | | | | 27 | | 19 | 22 | 81.6 |

(EXAMPLES 1-3)

The nonwoven fabric according to EXAMPLES 1 to 3 listed in TABLES 1 and 2 comprises the composite fibers 2 of 100% by weight. In TABLE 1, the composite fibers 2 are referred to as composite fibers I. Polyester (PET) having a fusion point of 260°C was used as the core component and high density polyethylene (PE) having a fusion point of 130°C was used as the sheath component in this composite fiber I. Tow from which the composite fibers I is to be obtained was coated with surfactant of 0.4% by weight as the lubricant treatment in the step IV of Fig. 2 and thereby the tow was modified to be hydrophilic. Then the tow was processed by the crimper to have mechanical crimps at a rate of 15/25 mm. The tow after having been crimped was subjected to a heat treatment at a temperature of 120°C for 7 min. The nonwoven fabric according to EXAMPLES 1 and 2 is planar as exemplarily shown in Fig. 1 which is obtained through the production process shown in Fig. 2 while the nonwoven fabric according to EXAMPLE 3 is similar to the nonwoven fabric exemplarily illustrated by Fig. 4 which is obtained through the production process of Fig. 5. It should be noted that the step IX in Fig. 2 was not used by EXAMPLES 1 and 3 and this step IX was used only in EXAMPLE 2. In EXAMPLE 3, a molding plate like the molding plate 220 illustrated by Fig. 6 was used, in which the perforated zones 221 and the imperforated zones 222 are alternately repeated in units of 5 mm circumferentially of the suction drum 210 and each of the perforated zones 221 is formed with through-holes 223 each having a diameter of 0.6 mm at an area ratio of 22%.

Figs. 11 and 12 exemplarily show a method for measurement of "average fiber angle θ and result thereof with respect to the nonwoven fabric according to EXAMPLE 1. Fig. 11 is a 30 times magnified photograph of a cut surface of the nonwoven fabric extending in parallel to the cross direction CD. On the photograph of Fig. 11, a primary vertical line L with respect to the nonwoven fabric and two auxiliary lines M, N extending on both sides of and in parallel to this primary vertical line L are drawn. Fig. 11 further includes a fiber angle measuring line D which passes through the intersection points between the primary vertical line L and the two auxiliary lines M, N. Fig. 12 also illustrates the primary vertical line L, the pair of auxiliary lines M, D and the fiber angle measuring line D together with a table listing smaller values of the respective pairs of intersection angles $\alpha$, $\beta$ measured on the fiber samples No. 1 to No. 48. "Average fiber angle θ " corresponding to an arithmetic average value of the values listed in Fig. 12 and was equal to 70.8°.

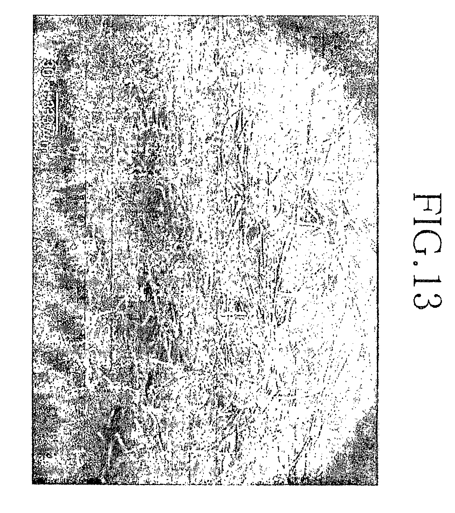

Fig. 13 is a photograph of a cut surface of the nonwoven fabric according to EXAMPLE 1 taken in parallel to the machine direction enlarged in the same magnification as in the case of Fig. 11. As will be apparent, most of the fibers extend in the machine direction with a gentle undulation.

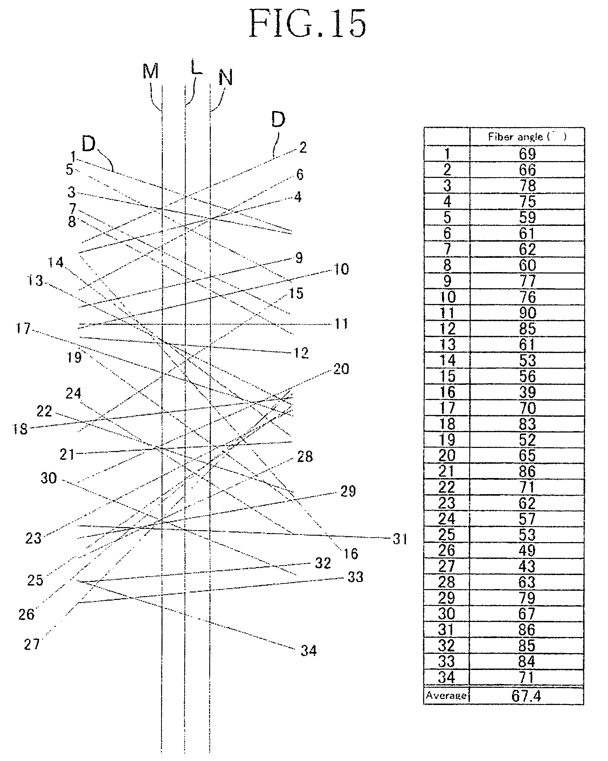

Figs. 14 and 15 exemplarily show a method for measurement of "average fiber angle θ and result thereof with respect to the nonwoven fabric according to EXAMPLE 3. Fig. 14 is a 30 times magnified photograph of one of the crests appearing in a cut surface of the nonwoven fabric extending in parallel to the cross direction CD. On the photograph of Fig. 14, a primary vertical line L passing through the apex of this crest and two auxiliary lines M, N extending on both sides of and in parallel to this primary vertical line L are drawn. Fig. 14 further includes a fiber angle measuring line D which passes through the intersection points between the primary vertical line L and the two auxiliary lines M, N. Fig. 15 also illustrates the primary vertical line L, the pair of auxiliary lines M, D and the fiber angle measuring line D of Fig. 14 together with a table listing smaller values of the respective pairs of intersection angles measured on the fiber samples No. 1 to No. 34. "Average fiber angle θ " is equal to 67.4°. It should be noted here that one example of the average fiber angle θ measured in the vicinity of the trough of the nonwoven fabric according to EXAMPLE 3 was equal to 59°.

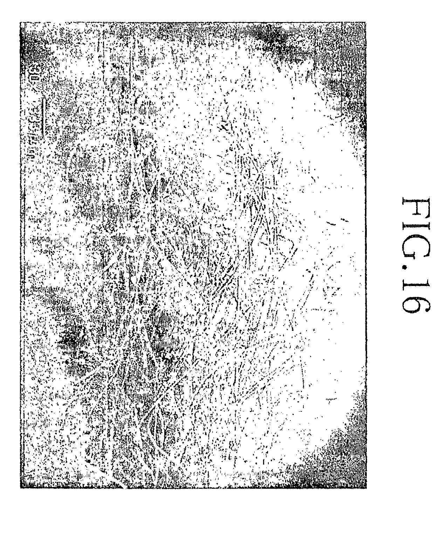

Fig. 16 is a photograph of a cut surface of the crest apex of the nonwoven fabric according to EXAMPLE 3 extending in parallel to the machine direction as observed in the same magnification as in the case of Fig. 14. Most of the fibers extend in the machine direction with a gentle undulation.

(EXAMPLES 4-6)

The nonwoven fabric according to EXAMPLES 4-6 comprises the composite fiber 2 of 100% and this composite fiber 2 comprises composite fiber I and composite fibers II mixed together at a weight ratio of 70:30 to 30:70. The composite fibers I each has a fiber length of 38 mm or 51 mm and has previously been, in the form of tow, mechanically crimped at a rate of 15/25 mm. The composite fibers II each has a fiber length of 51 mm and has previously been, in the form of tow, mechanically crimped at a rate of 18/25 mm. Both the composite fibers I and the composite fibers II have previously been subjected to a lubricant treatment. More specifically, the tow was coated with surfactant of 0.4% by weight adapted to modify it to become hydrophilic, then mechanically crimped and heat treated at a temperature of 120°C for 7 min.

(EXAMPLE 7)

The nonwoven fabric according to EXAMPLE 7 listed in TABLES 1 and 2 was made by using the web comprising the composite fibers I corresponding to the composite fibers 2 used in EXAMPLE 1 and the blending fibers 112 shown in the production process of Fig. 2 blended at a weight ratio of 50:50. The blending fibers 112 used in this EXAMPLE was obtained by coating the tow with the surfactant of 0.4% by weight to modify the tow to become hydrophilic, then mechanically crimping the tow at a rate of 15/25 mm, heat-treating the tow at a temperature of 90°C for 7 min and then cutting the tow treated in this manner into a length of 38 mm in the form of core-sheath type composite fiber.

(EXAMPLES 8-10)

The composite fibers I used in EXAMPLE 1 were used as the composite fibers 2 while the latently crimped core-sheath type composite fibers previously coated with surfactant of 0.4% by weight serving to modify the fibers to become hydrophilic and mechanically crimped at a rate of 15/25 mm was used as the blending fiber 112. The composite fibers I and the blending fibers 112 were blended together at a weight ratio of 80: 20 - 50:50. A crimping rate exhibited by the blending fibers 112 as it is heated was evaluated on the basis of a contraction ratio of the web.

(EXAMPLES 11 and 12)

The nonwoven fabric having the configuration exemplarily shown in Fig. 4 was made by adopting the production process exemplarily shown in Fig. 7 and a liquid-permeation rate was measured on the nonwoven fabric obtained. In EXAMPLE 11, the first card 301a in Fig. 7 provided the first web 302a as listed in TABLE 1. As will be apparent from TABLE 1, the first web 302a comprised the composite fibers I and the blending fibers at a ratio of 80:20 and had a basis weight of 10 g/m². This first web 302a was referred to as a bottom layer in TABLE 1. Similarly, the second card 301b provided the second web 302b as listed in TABLE 1. The second web 302b comprised the composite fibers I and the blending fibers at a ratio of 80:20 and had a basis weight of 10 g/m². This second web 302b was referred to as an intermediate layer in TABLE 1 and placed on the first web 302a. The third card 301c provided the third web 302c being same in composition as well as in basis weight as the second web 302b. The third web 302c was referred to as a top layer in TABLE 1 and placed on the second web 302b. The first, second and third webs 302a, 302b, 302c were placed one upon another to form the web laminate 302d which was, in turn, fed in the machine direction MD so that the first web 302a may be kept in contact with the suction drum 211 of the molding means 210. Operating condition subsequent to the molding means 210 was same as in EXAMPLE 3.

The nonwoven fabric according to EXAMPLE 12 is similar to the nonwoven fabric according to EXAMPLE 11 except that the first web 301a had a basis weight of 15 g/m².

(COMPARATIVE EXAMPLES 1 and 2)

The nonwoven fabrics according to COMPARATIVE EXAMPLES 1 and 2 are similar to the nonwoven fabric according to EXAMPLE 1 except the condition under which the tow is heat-treated. Specifically, the mechanically crimped tow was heat-treated at a temperature of 90°C for 7 min in the case of COMPARATIVE EXAMPLE 1 and at a temperature of 100°C for 7 min in the case of COMPARATIVE EXAMPLE 2. The remaining conditions for production of the nonwoven fabrics were same as those in EXAMPLE 1.

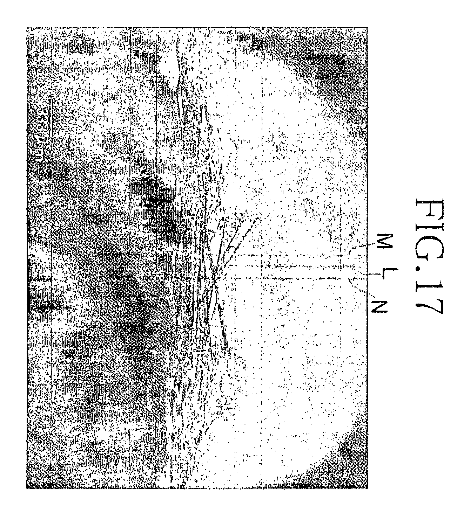

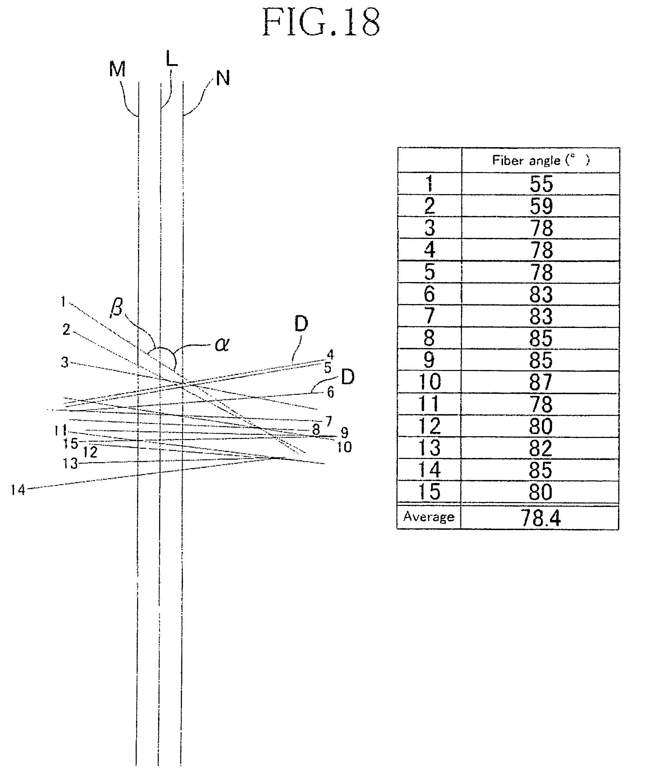

Figs. 17 and 18 exemplarily illustrate, in the same manner as Figs. 11 and 12, a method for measurement of "average fiber angle θ and result thereof with respect to the nonwoven fabric according to COMPARATIVE EXAMPLE 1. Fig. 17 is a 30 times magnified photograph of a cut surface of the nonwoven fabric extending in parallel to the cross direction CD. Referring to Fig. 17, in a region of the cut surface having a typical configuration without any fibers extraordinarily projecting from the surface of the nonwoven fabric, a primary vertical line L and two auxiliary lines M, N extending on both sides of and in parallel to this primary vertical line L are drawn. Fig. 17 further includes a fiber angle measuring line D used for respective fibers to be measured. Fig. 18 also illustrates the primary vertical line L, the pair of auxiliary lines M, D and the fiber angle measuring line D together with a table listing smaller values of the respective pairs of intersection angles $\alpha$, $\beta$ measured on the fiber samples No. 1 to No. 15. "Average fiber angle θ" of the nonwoven fabric according to COMPARATIVE EXAMPLE 1 was 78.4°.

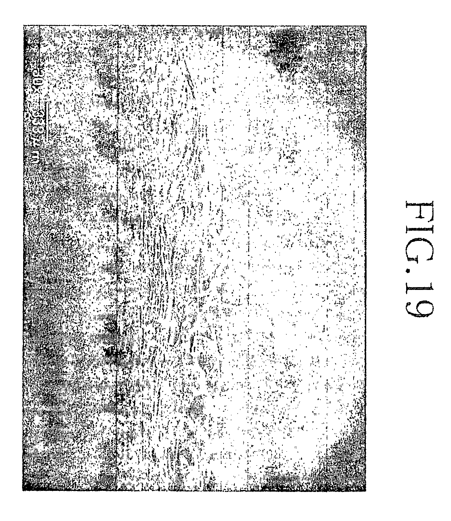

Fig. 19 is a photograph of a cut surface of the nonwoven fabric according to COMPARATIVE EXAMPLE 1 taken in parallel to the machine direction and observed with the same magnification as in the case of Fig. 17. As will be apparent from this photograph, the fibers flatly extend in the machine direction and are close together in the thickness direction.

In COMPARATIVE EXAMPLES 3 and 4, a content of the composite fiber in the nonwoven fabric was 20% by weight or 10% by weight, the average fiber angle θ was 75° or higher, a first liquid-permeation rate was longer than 15 sec and a secondary liquid-permeation rate was longer than 20 sec.

As is suggested by TABLES 1 and 2, a temperature for heat treatment of the tow after it has been mechanically crimped, i.e., a heat treatment temperature after crimping listed in TABLE 1 may be adjusted approximately to a fusion temperature of the low fusion point resin forming the sheath component of the composite fibers, preferably to a range defined between this fusion point and a temperature 20°C lower than this fusion point to minimize a deformation residual ratio and thereby to maximize an elastic recover of the tow after compression. This was true for the web as well as the nonwoven fabric obtained from such tow and well reflected on a bulk retention ratio of the web and a specific volume of the nonwoven fabric. As will be easily understood from values of the fusion heat quantity ΔH of the tow listed in TABLE 2, increase in the temperature at which the tow is heat-treated resulted in increase of the fusion heat quantity ΔH. The fusion heat quantity ΔH referred to herein should be understood to be the fusion heat quantity ΔH of the low fusion point resin in the composite fiber used in EXAMPLES 1 and 2 as well as in COMPARATIVE EXAMPLES 1 and 2, i.e., the fusion heat quantity ΔH of polyethylene and such increase in the fusion heat quantity ΔH is believed to mean that the low fusion point resin in EXAMPLES has a thermal stability higher than the low fusion point resin in COMPARATIVE EXAMPLES can have and consequentially the tow as well as staple derived therefrom is able to maintain the crimped state even under heating. The nonwoven fabric according to EXAMPLES is also characterized by its average fiber angle θ of 75° or less, which is smaller than the average fiber angle θ of the nonwoven fabric according to COMPARATIVE EXAMPLES. In other words, in the cut surface extending in parallel to the cross direction CD of the nonwoven fabric placed on the horizontal plane, both the composite fibers 2 and the blending fibers 112 tend to extending in the vertical direction rather than tending to extend flatly in the horizontal direction. Such average fiber angle θ is reflected on the liquid-permeation rate of the  nonwoven fabric according to EXAMPLES. Specifically, the liquid-permeation rate of the primarily dropped artificial menstrual blood was 15 sec or shorter and the liquid-permeation rate of secondarily dropped artificial menstrual blood was 20 sec or shorter. In the nonwoven fabric according to COMPARATIVE EXAMPLES, the average fiber angle θ exceeded 75° and consequentially the liquid-permeation rate of primarily dropped artificial menstrual blood exceeded 15 sec and the liquid-permeation rate of secondarily dropped artificial menstrual blood exceeds 20 sec. In contrast with the nonwoven fabric according to COMPARATIVE EXAMPLES, the nonwoven fabric according to EXAMPLES can reliably maintain its ability to spot-absorb body fluids repetitively discharged.

According to EXAMPLE 7, the mechanically crimped composite fiber used in COMPARATIVE EXAMPLE 1may used as the blending fiber 112.

According to EXAMPLES 8-12, the latently crimped composite fiber may be used as the blending fiber 112.

According to EXAMPLES 11 and 12, a plurality of webs obtained from a plurality of cards may be placed one upon  another to form the web laminate and the nonwoven fabric according to the invention may be obtained using this web laminate.

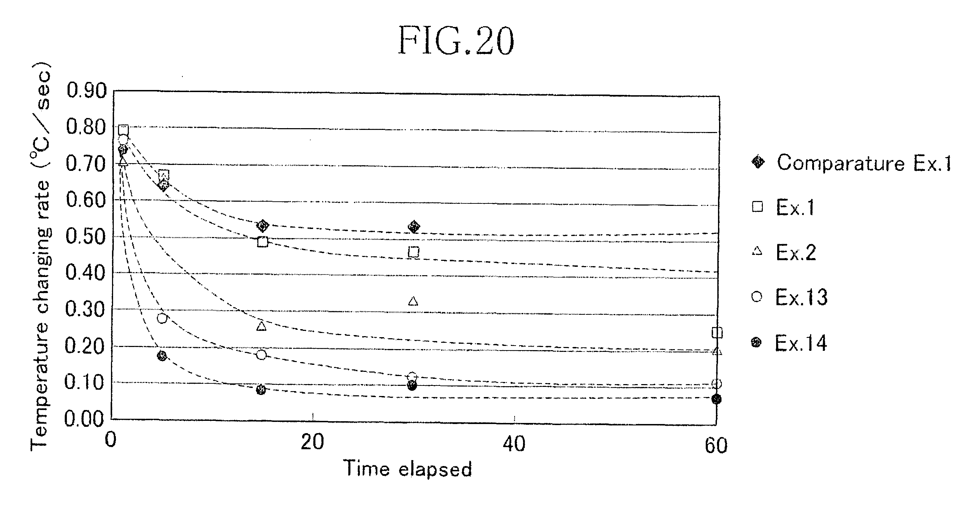

Fig. 20 is a graphic diagram plotting a result of a performance comparison test for the nonwoven fabric according to EXAMPLES 3, 13 and 14 and the nonwoven fabric according to COMPARATIVE EXAMPLE 1 used as the liquid-pervious topsheet 251 of the sanitary napkin 250 illustrated by Fig. 10. In this test, the sanitary napkin 250 using the nonwoven fabric according to the EXAMPLES or COMPARATIVE EXAMPLES to be tested was put in a laboratory at a temperature of 20°C and a relative humidity of 60%. 6 me of artificial menstrual blood (See paragraph [12. Liquid permeation rate]) at a temperature of 20°C was dropped onto the nonwoven fabric and a sensor of the measuring device FINGER ROBOT THERMO LAB (manufactured by KATO TECH CO., LTD. , Minami-Ku, Kyoto City) was applied to the region in which the artificial menstrual blood has been dropped. A temperature changing rate (°C/sec) indicated by the sensor was recorded via steps as will be described below:

Step 1: topsheet of commercially available sanitary napkin (Trade Name: SOFY FUWAPITASURIMU 25 mm manufactured by  Uni-Charm Corporation) is replaced by the nonwoven fabric under test to prepare the sanitary napkin to be tested.

Step 2: the sensor is set to 37°C.

Step 3 : an acrylic plate having a thickness of 13 mm and formed with a rectangular through-hole of 40 $\times$ 10 mm is placed upon the nonwoven fabric for test in the sanitary napkin.

Step 4: 6 m$\ell$ of artificial menstrual blood at a temperature of 20°C is dropped into the through-hole of the acrylic plate.

Step 5: upon disappearance of artificial menstrual blood from the surface of the nonwoven fabric for test, the sensor is pressed against the artificial menstrual blood dropped region to record the temperature changing rate as indicated by the sensor. The sensor is controllably pressed against the nonwoven fabric for test so that a surface pressure of 20 to 30 gf/cm$^2$ may be achieved.

Step 6: the temperature changing rates at 1, 5, 15, 30 and 60 sec after the measurement has been started.

Referring to Fig. 20, while the temperature changing rate is generally tends to get late as a time elapses, the temperature changing rate the nonwoven fabric according to EXAMPLES tends to get late after the measurement has been  started more rapidly than in the case of the nonwoven fabric according to COMPARATIVE EXAMPLES. Such behavior is remarkable in the nonwoven fabric according to EXAMPLES 3, 11 and 12 which is formed with the crests 202 and the troughs 203 exemplarily illustrated by Fig. 4.

The inventors have found that, between a cold sense experienced by the human finger at a moment that the finger comes in contact with the object to be measured by FINGER ROBOT THERMO LAB and a temperature changing rate indicated by the sensor, relationships as follow are established:

No cold sense is experienced at a temperature changing rate of 0 to 0.30°C/sec;

A certain degree of cold sense is experienced at a temperature changing rate of 0.30 to 0.50°C/sec; and

A definite cold sense is experienced at a temperature changing rate of 0.50°C /sec.

This finding suggests that, in the case of the sanitary napkin using the nonwoven fabric according to EXAMPLES, particularly according to EXAMPLES 3, 11 and 12, the temperature changing rate will decrease to 0.30°C or lower within 30 sec after the measurement has been started. Consequentially, the wearer of such sanitary napkin will experience feeling of incompatibility due to a cold sense merely for a short time. Generally, upon discharge of menstrual blood, the wearer experiences such feeling of incompatibility due to the cold sense and simultaneously immobilizes herself or slows down a movement of her body to minimize leak of menstrual blood and/or contamination of her skin therewith. The sanitary napkin using the nonwoven fabric according to EXAMPLES 3, 11 and 12 not only allows menstrual blood to be rapidly absorbed but also allows any feeling of incompatibility to disappear in a short time. Therefore it is no more necessary for the wearer to immobilize herself or to slow down movement of her body.

A temperature changing rate when the sensor of FINGER ROBOT THERMO LAB is put in contact with artificial menstrual blood at a temperature of 20°C was 0.80°/sec and a temperature changing rate when the sensor is put in contact with the nonwoven fabric for test before artificial menstrual blood is dropped onto the nonwoven fabric was 0.04°C.

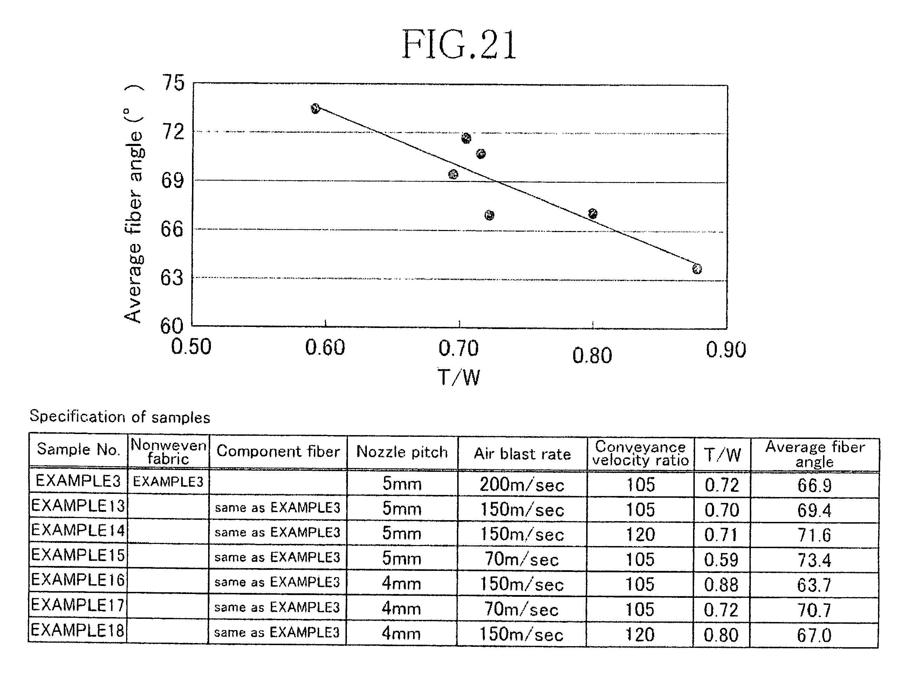

Fig. 21 is a graphic diagram showing a relationship between the value of T/W (See Fig. 9) of the crest and the average fiber angle θ in the nonwoven fabric according to EXAMPLE 3. Fig. 21 further shows a relationship between the value of T/W of the crest and the average fiber angle θ in the nonwoven fabrics according to EXAMPLES 13 to 18 each comprising the same fiber components as those of the nonwoven fabric according to EXAMPLE 3 but obtained by the production process differing from the production process for EXAMPLE 3 with respect to the pitch of the individual nozzles 215 in the respective nozzle assemblies 212, 213, 214 as well as with respect to the conveyance velocity ratio between the step of fusing together (step X) and the step of reeling (step XI).

The value of T/W may be measured by executing steps as will be described below in this order.

(1) The nonwoven fabric used as a sample for measurement is heated at a temperature of 70°C for 30 min to remove any traces of folding in the course of handling and thereby to flatten the sample.
(2) Substitute edge HA-100 for KOKUYO's cutter knife is used to cut the sample in the cross direction CD to prepare a cut surface for observation extending in parallel to the cross direction CD.
(3) With the sample placed on a horizontal plane, the cut surface is observed through digital microscope an VHX-900 manufactured by Keyence Corporation and a macro photograph of the cut surface x 25 is taken.
(4) On the macro photograph, a vertical line with respect to a horizontal line defined by the horizontal plate surface so as to extending through the crest's apex of the sample. A distance from the reference line to the apex is measured to obtain a height T of the crest (See Fig. 9). The horizontal line passing through a point lying at 1/2 of the height T in parallel to the reference line is drawn and a width of the crest along this horizontal line is measured to obtain a width W of the crest. In this way, a value T/W is calculated.
(5) When the vertical line passing the apex is drawn, the crest presenting an average shape is selected for measurement. The crest to be selected for measurement has its apex preferably free from any fibers extraordinarily projecting therefrom.

As will be apparent from Fig. 21, when it is desired to obtain the nonwoven fabric of Fig. 4 presenting the average fiber angle of 75° or less, the value of T/W in the crest is preferably limited to a range of 0.55 to 1.00.

## Claims

1. A liquid-pervious nonwoven fabric having a basis weight in a range of 10 to 200 g/m<2>, said liquid-pervious nonwoven fabric having a first direction, a second direction orthogonal to said first direction and a thickness direction in orthogonal relationship one to another, said nonwoven fabric comprising core-sheath type composite fibers of 100 to 30% by weight as composite fibers and thermoplastic synthetic fibers of 0 to 70% by weight as blending fibers for said composite fibers, said core-sheath type composite fibers comprising core and sheath components in concentric relationship with each other wherein a thermoplastic synthetic resin forming said sheath component has a fusion point lower than a fusion point of a thermoplastic synthetic resin forming said core component, said liquid-pervious nonwoven fabric further comprising:

said composite fibers have a fineness of 1 to 17 dtex and a fiber length of 10 to 150 mm, said composite fibers extending in said first direction with curvatures repeated in said thickness direction as viewed in a cut surface of said nonwoven fabric taken in parallel to said first direction and extending in said thickness direction as viewed in a cut surface of said nonwoven fabric taken in parallel to said second direction so that the composite fibers may intersect together among them and/or the blending fibers and, at respective points of intersection, said composite fibers are fused together with themselves and/or with the blending fibers as said low fusion point resin is fused; and
when said nonwoven fabric is placed in a horizontal plane, some of said composite fibers and said blending fibers intersect, in said cut surface of said nonwoven fabric taken in parallel to said second direction, a vertical line with respect to said horizontal plane at acute angles inclusive of 90[deg.] and some of said composite fibers and said blending fibers intersect said vertical line at obtuse angles larger than 90[deg.] so that an average fiber angle corresponding to an average value of said acute intersection angles is 75[deg.] or less and
wherein said nonwoven fabric has upper and lower surfaces opposed to each other in said thickness direction and said upper surface is formed with a plurality of crests extending in parallel to said first direction and a plurality of troughs each extending in said first direction between each pair of adjacent said crests.

2. The nonwoven fabric as recited by Claim 1, wherein said composite fibers contain among them spirally crimped thermoplastic synthetic fibers at content up to 50% by weight.

3. The nonwoven fabric as recited by Claim 1 or 2, wherein at least one of hydrophilic blending natural fibers and hydrophilic blending semi-synthetic fibers is contained in said nonwoven fabric up to 10% by weight with respect to total weight of said nonwoven fabric.

4. The nonwoven fabric as recited by any one of Claims 1 through 3, wherein one of said composite fibers and said blending thermoplastic synthetic fibers has its surface modified to be hydrophilic.

5. The nonwoven fabric as recited by Claim 4, wherein, with said lower surface of said nonwoven fabric placed in the horizontal plane, said average fiber angle defined between said vertical line extending through an apex of said crest and said composite fibers and/or said blending fibers is 75[deg.] or less.

6. The nonwoven fabric as recited by any one of Claims 1 through 5, wherein said nonwoven fabric is used as a top sheet in a sanitary napkin.

7. A method for making liquid-pervious nonwoven fabric including having a basis weight In a range of 10 to 200 g/m<2>, said liquid-pervious nonwoven fabric having a mechanical direction and a cross direction in orthogonal relationship to each other and including core-sheath type composite fibers of 100 to 30% by weight as composite fibers, wherein said core-sheath type composite fibers comprising core and sheath components in concentric relationship with each other and a thermoplastic synthetic resin forming said sheath component has a fusion point lower than a fusion point of a thermoplastic synthetic resin forming said core component, said method comprising the steps:

    a. forming said core-sheath type composite fibers followed by obtaining a tow from a plurality of said core-sheath type composite fibers and then drawing said tow;
    b. mechanically crimping said tow having been stretched in the step (a) so as to repeat curvatures in a longitudinal direction of said tow;
    c. subjecting said tow having been crimped in the step (b) to an annealing treatment;
    d. cutting said tow having been subjected to said annealing treatment in the step (c) into a length of 10 to 150 mm so as to obtain an assembly of said composite fibers in the form of staples;
    e. fibrillating the assembly of said composite fibers through a card to obtain a web comprising said composite fibers and having a desired basis weight;
    f. heating said web to fuse said resin of low fusion point and thereby fusing together said composite fibers in said web one at intersection points thereof; and
    g. cooling said web after said step (f)
    wherein said annealing treatment in said step (c) is carried out at a temperature between the fusion temperature of said low fusion point resin forming said sheath component and a temperature 20[deg.]C lower than said fusion temperature.

8. The method as recited by Claim 7, further including a step of arranging a plurality of said cards in said machine direction followed by placing said webs obtained from respective said cards one upon another to form laminate web so as
to be treated as said web in a step subsequent to said step (f).

9. The method as recited by Claim 7 or 8, further including, between said step(e) and said step(f), a step of preheating said web after said composite fibers have been fused together at the intersection points thereof and before said web is conveyed to said step (f).

10. The method as recited by any one of Claims 7 through 9, said step (f) includes a sub-step of compressing said web in said thickness direction using compressed air or mechanical means to increase a density of said web and a sub-step of fusing said composite fibers in said web together at the intersection points of said composite fibers.

11. The method as recited by Claim 9 or 10, said step of preheating described in Claim 10 includes a sub-step of emitting a jet of heated and pressurized air from a plurality of individual nozzles arranged in said cross direction to said web conveyed on support means in said machine direction to form said web with a plurality of crests parallel extending in said machine direction and a plurality of troughs each defined between a pair of adjacent said crests and extending in said machine direction.

**12.** The method as recited by any one of Claims 7 through 11, said step (e) includes a sub-step of adding thermoplastic synthetic fiber having latent crimps as blending fibers to said composite fibers so as to occupy 0 to 50% by weight with respect to total weight of said nonwoven fabric.

**13.** The method as recited by Claim 11 or 12, wherein a ratio of thickness T of said nonwoven fabric measured in a region inclusive of an apex of said crest in a cut surface of said nonwoven fabric taken in parallel to said cross direction to a width W of said crest measured at a level corresponding to 1/2 of said thickness T is in a range of 0.55 to 1.00.

**14.** The method as recited by any one of Claims 7 through 13, said step (b) comprises a step of feeding said tow into a box-type crimper so that said composite fibers may be mechanically crimped in zigzags at a rate of 10 to 35 crimps/ 25 mm.

**15.** The method as recited by any one of Claims 7 through 11 and 13 through 14, wherein said composite fibers intersect, in said cut surface of said nonwoven fabric taken in parallel to said cross direction, a vertical line with respect to said horizontal plane at acute angles inclusive of 90[deg.] and some of said composite fibers and said blending fibers intersect said vertical line at obtuse angles larger than 90[deg.] so that an average fiber angle corresponding to an average value of said acute intersection angles may be 75[deg.] or less.

**16.** The method as recited by any one of Claims 12 through 14, wherein said composite fibers and said thermoplastic synthetic fibers used as said blending fibers intersect, in said cut surface of said nonwoven fabric taken in parallel to said cross direction, a vertical line with respect to said horizontal plane at acute angles inclusive of 90[deg.] and some of said composite fibers and said blending fibers intersect said vertical line at obtuse angles larger than 90 [deg.] so that an average fiber angle corresponding to an average value of said acute intersection angles may be 75[deg.] or less.

**17.** The method as recited by any one of Claims 7 through 16, said step (e) comprises a sub-step of blending at least one of hydrophilic natural fibers and hydrophilic semi-synthetic fibers in said nonwoven fabric so that a content of said hydrophilic natural fiber or said hydrophilic semi-synthetic fibers are 0 to 10% by weight with respect to total weight of said nonwoven fabric.

**Patentansprüche**

**1.** Flüssigkeitsdurchlässiger Vliesstoff mit einem Flächengewicht in einem Bereich von 10 bis 200 g/m<2>, wobei der flüssigkeitsdurchlässige Vliesstoff eine erste Richtung, eine zweite Richtung orthogonal zur ersten Richtung und eine zu diesen orthogonale Dickenrichtung aufweist, wobei der Vliesstoff Kern-Hülle-Verbundfasern aus 100 bis 30 Gew.% als Verbundfasern und thermoplastische Synthetikfasern aus 0 bis 70 Gew.% als Mischfasern für die Verbundfasern umfasst, wobei die Kern-Hülle-Verbundfasern zueinander konzentrische Kern- und Hüllebestandteile umfassen,

wobei ein den Hüllebestandteil bildendes thermoplastisches synthetisches Harz einen Schmelzpunkt aufweist, der niedriger ist als ein Schmelzpunkt des den Kernbestandteil bildenden thermoplastischen synthetischen Harzes, wobei der flüssigkeitsdurchlässige Vliesstoff ferner umfasst:

die Verbundfasern weisen eine Feinheit von 1 bis 17 dtex und einer Faserlänge von 10 bis 150 mm auf, wobei die Verbundfasern sich in der ersten Richtung mit in Dickenrichtung sich wiederholenden Krümmungen, gesehen in einer Schnittfläche des Vliesstoffes parallel zu der ersten Richtung, erstrecken und

sich in der Dickenrichtung, gesehen in einer Schnittfläche des Vliesstoffes parallel zu der zweiten Richtung, erstrecken, so dass sich die Verbundfasern untereinander und/oder mit den Mischfasern überkreuzen können und die Verbundfasern zusammen mit sich selbst an jeweiligen Kreuzungspunkten und/oder mit den Mischfasern verschmolzen sind, wenn das Harz mit niedrigem Schmelzpunkt geschmolzen wird, und

wenn der Vliesstoff in eine horizontalen Ebene gelegt wird schneiden einige der Verbundfasern und der Mischfasern in der Schnittfläche des Vliesstoffs parallel zu der zweiten Richtung eine vertikale Linie in Bezug auf die horizontale Ebene in einem spitzen Winkel einschließlich 90 Grad und einige der Verbundfasern und der Mischfasern schneiden die vertikale Linie in einem stumpfen Winkel größer als 90 Grad, so dass ein durchschnittlicher Faserwinkel, der einem mittleren Wert der spitzen Schnittwinkel entspricht, 75 Grad oder weniger ist, und wobei das Faservlies obere und untere Flächen aufweist, die einander in der Dickenrichtung gegenüber liegen, und die obere Fläche mit einer Vielzahl von sich parallel zu der ersten Richtung erstreckenden Erhebungen und einer Vielzahl von Vertiefungen, die sich jeweils in der ersten Richtung zwischen jedem Paar benachbarter Erhebungen erstrecken, gebildet ist.

2. Vliesstoff nach Anspruch 1, wobei unter den Verbundfasern spiralförmig gekräuselte thermoplastische synthetische Fasern mit einem Anteil von bis zu 50 Gew.% enthalten sind.

3. Vliesstoff nach Anspruch 1 oder 2, wobei zumindest eine der natürlichen hydrophilen Mischfasern und der semi-synthetischen hydrophilen Mischfasern in dem Vliesstoff mit bis zu 10 Gew.% bezogen auf das Gesamtgewicht des Vliesstoffs enthalten sind.

4. Vliesstoff nach einem der Ansprüche 1 bis 3, wobei eine der Oberflächen der Verbundfasern oder der thermoplastischen Synthetikmischfasern modifiziert wurde, hydrophil zu sein.

5. Vliesstoff nach Anspruch 4, wobei, wenn die untere Fläche des Vliesstoffs in der horizontalen Ebene angeordnet ist, der durchschnittliche Faserwinkel, bestimmt zwischen der senkrechten, sich durch einen Scheitel der Erhebung erstreckenden Linie und den Verbundfasern und/oder den Mischfasern, 75 Grad oder weniger ist.

6. Vliesstoff nach einem der Ansprüche 1 bis 3, wobei das Vlies als eine Oberlage einer Damenbinde verwendet wird.

7. Verfahren zum Herstellen eines flüssigkeitsdurchlässigen Vliesstoffs mit einem Flächengewicht in einem Bereich von 10 bis 200 g/m$^2$, wobei der flüssigkeitsdurchlässige Vliesstoff eine Maschinenrichtung und eine Querrichtung aufweist, die jeweils zueinander orthogonal sind, und mit Kern-Hülle-Verbundfasern mit 100 bis 30 Gew.% als Verbundfasern, wobei die Kern-Hülle-Verbundfasern zueinander konzentrische Kern- und Hüllebestandteile umfassen, wobei ein den Hüllebestandteil bildendes thermoplastisches synthetisches Harz einen Schmelzpunkt aufweist, der niedriger ist ein Schmelzpunkt des den Kernbestandteil bildenden thermoplastischen synthetischen Harzes, wobei das Verfahren ferner die Schritte umfasst:

   a) Bilden der Kern-Hülle-Verbundfasern gefolgt von Bilden eines Stranges aus einer Vielzahl von Kern-Hülle-Verbundfasern und anschließendes Ziehen des Stranges;
   b) Mechanisches Kräuseln des in Schritt a) gezogenen Stranges, um Krümmungen in der Längsrichtung des Stranges zu wiederholen;
   c) Unterziehen des in dem Schritt b) gekräuselten Stranges einer Glühbehandlung;
   d) Schneiden des in Schritt c) einer Glühbehandlung unterzogenen Stranges in einer Länge von 10 bis 150 mm, um eine Anordnung der Verbundfasern in Form von Stapeln zu erhalten;
   e) Fibrillieren der Anordnung der Verbundfasern durch eine Fibrillier-Maschine (engl. "card"), um eine Lage mit den Verbundfasern und mit einem gewünschten Flächengewicht zu erhalten;
   f) Erhitzen der Lage zum Schmelzen des Harzes mit niedrigem Schmelzpunkt und dadurch miteinander Verschmelzen der Verbundfasern in der Lage an ihren Schnittpunkten; und
   g) Abkühlen der Bahn nach dem Schritt (f),
   wobei die Glühbehandlung in dem Schritt (c) bei einer Temperatur zwischen der Schmelztemperatur des Harzes mit niedrigem Schmelzpunkt, das den Hüllenbestandteil bildet, und einer Temperatur von 20 Grad Celsius unterhalb der Schmelztemperatur durchgeführt wird.

8. Verfahren nach Anspruch 7, das ferner einen Schritt des Anordnens einer Mehrzahl der Fibrillier-Maschinen in der Maschinenrichtung umfasst, gefolgt von einem Aufeinanderlegen der durch die jeweils von den Fibrillier-Maschinen erhaltenen Lagen, um ein Laminat zu bilden, um in einem dem Schritt f) nachfolgenden Schritt als Lage behandelt zu werden.

9. Verfahren nach Anspruch 8 oder 9, ferner zwischen dem Schritt e) und dem Schritt f) umfassend einen Schritt des Vorheizens der Lage nachdem die Verbundfasern an ihren Schnittpunkten zusammengeschmolzen wurden und bevor die Lage zu dem Schritt f) befördert wird.

10. Verfahren nach mindestens einem der Ansprüche 7 bis 9, wobei der Schritt f) einen Unterschritt des Komprimierens der Lage in der Dickenrichtung unter Nutzung von Druckluft oder mechanischen Mitteln, um eine Dichte der Lage zu erhöhen, und einen Unterschritt des Zusammenschmelzens der Verbundfasern in der Lage an den Kreuzungspunkten der Verbundfasern, umfasst.

11. Verfahren nach Anspruch 9 oder 10, wobei der in Anspruch 10 beschriebene Schritt des Vorheizens, einen Unterschritt des Emittierens eines Strahls erwärmter und unter Druck stehender Luft aus einer Vielzahl von einzelnen Düsen umfasst, die in Querrichtung zu der Lage angeordnet sind und auf Tragmitteln in der Maschinenrichtung befördert werden, um die Lage mit einer Vielzahl von sich in der Maschinenrichtung parallel erstreckenden Erhebungen und einer Vielzahl von Vertiefungen, die jeweils zwischen einem Paar benachbarten Erhebungen bestimmt sind und sich in der Maschinenrichtung erstrecken, zu formen.

12. Verfahren nach mindestens einem der Ansprüche 7 bis 11, wobei der Schritt e) einen Unterschritt des Zugebens von thermoplastischen synthetischen Fasern mit verborgener Kräuselung als Mischfasern zu den Verbundfasern, um 0 bis 50 Gew.% bezüglich des Gesamtgewichts des Vliesstoffes einzunehmen.

13. Verfahren nach Anspruch 11 oder 12, wobei ein Verhältnis der Dicke T des Vliesstoffs, gemessen in einem Bereich einschließlich einer Spitze der Erhöhung in einer Schnittfläche des Vliesstoffs parallel zu der Querrichtung, zu einer Breite W der Erhöhung, gemessen auf einer Höhe entsprechend 1/2 der Dicke T, in einem Bereich von 0,55 bis 1,00 liegt.

14. Verfahren nach mindestens einem der Ansprüche 7 bis 13, wobei der Schritt b) einen Schritt des Zuführens des Stranges in einen Kräuseler nach Art einer Box umfasst, so dass die Verbundfasern mechanisch in ein Zickzack mit 10 bis 35 Kräuselungen/25 mm gekräuselt werden.

15. Verfahren nach mindestens einem der Ansprüche 7 bis 11 und 13 bis 14, wobei die Verbundfasern in der Schnittfläche des Vliesstoffs parallel zu der Querrichtung eine vertikale Linie in Bezug auf die horizontale Ebene in einem spitzen Winkel einschließlich 90 Grad schneiden und einige der Verbundfasern und der Mischfasern schneiden die vertikale Linie in einem stumpfen Winkel größer als 90 Grad, so dass ein durchschnittlicher Faserwinkel, der einem mittleren Wert der spitzen Schnittwinkel entspricht, 75 Grad oder weniger ist.

16. Verfahren nach mindestens einem der Ansprüche 12 bis 14, wobei die Verbundfasern und die als Mischfasern verwendeten thermoplastischen Synthetikfasern in der Schnittfläche des Vliesstoffs parallel zu der Querrichtung eine vertikale Linie in Bezug auf die horizontale Ebene in einem spitzen Winkel einschließlich 90 Grad schneiden und einige der Verbundfasern und der Mischfasern schneiden die vertikale Linie in einem stumpfen Winkel größer als 90 Grad, so dass ein durchschnittlicher Faserwinkel, der einem mittleren Wert der spitzen Schnittwinkel entspricht, 75 Grad oder weniger ist.

17. Verfahren nach mindestens einem der Ansprüche 7 bis 16, wobei der Schritt e) einen Unterschritt des Mischens von zumindest einer der natürlichen hydrophilen Fasern und hydrophilen semi-synthetischen Fasern in den Vliesstoff umfasst, so dass der Gehalt an hydrophilen natürlichen Fasern oder hydrophilen semi-synthetischen Fasern 0 bis 10 Gew.% bezogen auf das Gesamtgewicht des Vliesstoffs ist.

**Revendications**

1. Tissu non tissé perméable au liquide ayant un grammage dans une gamme de 10 à 200 g/m<2>, dans lequel le tissu non tissée perméable aux liquides, ayant une première direction, une deuxième direction orthogonale à la première direction et une direction de l'épaisseur orthogonale à la deuxième direction, dans lequel le tissu non-tissé comprend 100 à 30% en poids de fibres composites essentielles présentant une structure de noyau et de gaine (core-shell) et 0 à 70% en poids des fibres synthétiques thermoplastiques comme fibres de mélange pour que les fibres composites, ledit fibres composites essentielles ayant une structure des composants de noyau et de gaine mutuellement concentrique

dans lequel un composant de la résine synthétique thermoplastique formant la gaine a un point de fusion qui est inférieure à un point de fusion du composant de la résine synthétique thermoplastique formant le noyau, ledit tissu non-tissé perméable aux liquides, comprenant en outre:

les fibres composites ont une finesse de 1 à 17 dtex et une longueur de fibre de 10 à 150 mm, lesdites fibres composites s'étendant dans la première direction avec les courbures répétitives dans la direction d'épaisseur vu dans une plan de coupe du tissu non tissée parallèle à la première direction, et s'étendant dans la direction d'épaisseur vu dans une plan de coupe du tissu non tissée parallèle à la deuxième direction, de telle sorte que les fibres composites peuvent se croiser avec les uns aux autres et/ou avec les fibres mélangées et les fibres composites sont fusionnés avec elle-même en des points de croisement respectifs et/ou avec avec des fibres mélangées dès que la résine avec le point de fusion bas est fondue, et

si le tissu non-tissé est placé dans un plan horizontal une partie des fibres composites et des fibres de mélange coupant une ligne verticale par rapport au plan horizontal sous un angle aigu compris de 90 degrés dans le plan de coupe du tisse non-tissée en parallèle à la deuxième direction, et certains des fibres composites et des fibres mélangées se coupent la ligne verticale sous un angle obtus supérieur à 90 degrés, de sorte qu'un angle de fibre moyenne correspondant à une valeur moyenne de l'angle d'intersection aigu est de 75 degrés ou moins, et

dans lequel le tissu non-tissé comprend des surfaces supérieure et inférieure qui se font face de l'autre par rapport à la direction de l'épaisseur, et

la surface supérieure est formées d'une pluralité d'élévations s'étendent parallèlement dans la première direction et une pluralité de cavités, chacune s'étendant dans la première direction entre chaque paire d'élévations adjacentes.

2. Tissu non-tissé selon la revendication 1, dans lequel les fibres composites sont contenues de fibres synthétiques thermoplastiques frisées de manière hélicoïdale dans une proportion jusqu'à 50% en poids.

3. Tissu non-tissé selon la revendication 1 ou 2, dans lequel au moins un mélange de fibres naturelles hydrophiles et des fibres mélangées semi-synthétiques hydrophiles sont contenues dans le tissu non-tissé jusqu'à 10% en poids par rapport au poids total du tissu non-tissé.

4. Tissu non-tissé selon l'une quelconque des revendications 1 à 3, dans lequel l'une des surfaces des fibres composites ou des fibres mélangées synthétiques thermoplastiques ont été modifiés pour être hydrophiles.

5. Tissu non-tissé selon la revendication 4, dans lequel l'angle de fibre moyenne est 75 degrés ou moins qui est définie entre la ligne verticale s'étendant à travers un sommet d'élévations et des fibres composites et/ou de fibres mélangées, lorsque la surface inférieure du tissu non-tissé est disposé dans le plan horizontal.

6. Tissu non-tissé suivant l'une quelconque des revendications 1 à 3, dans lequel le tissu non-tissé est utilisé comme une feuille de dessus d'une serviette hygiénique.

7. Procédé de fabrication d'un tissu non-tissé perméable aux liquides ayant un grammage compris dans une gamme de 10 à 200 g/m<2>, dans lequel le tissu non-tissé perméable aux liquides ayant une direction de la machine et une direction transversale, chacun perpendiculaire l'une à l'autre, et avec 100 à 30% en poids des fibres composites essentielles présentant une structure de noyau et de gaine, lesdites fibres composites ayant une structure des composants de noyau et de gaine mutuellement concentrique dans lequel un composant de la résine synthétique thermoplastique formant la gaine a un point de fusion qui est inférieure à un point de fusion du composant de la résine synthétique thermoplastique formant le noyau, ledit procédé comprenant en outre les étapes consistant à

a) la formation des fibres composites présentant une structure de noyau et de gaine, suivie par la formation d'un brin d'une pluralité de fibres composites de type noyau-gaine et puis en tirant le brin;

b) le sertissage mécanique du brin établie dans l'étape a) pour répéter des courbures dans la direction longitudinale du brin;

c) soumettre le brin sertissé par l'étape b) à une traitement de recuit ;

d) couper le brin recuit dans l'étape c) à une longueur de 10 à 150 mm pour obtenir un assemblage de fibres composites sous forme de piles;

e) fibriller l'ensemble des fibres composites au moyen d'une machine fibrillation (en anglais "card") pour obtenir une couche avec des fibres composites et ayant un grammage souhaité;

f) le chauffage de la couche pour faire fondre la résine ayant un point de fusion bas et pour fusionner les fibres composites les uns aux autres dans une position au niveau de leurs intersections, et

g) le refroidissement de la bande après l'étape (f)

dans lequel ledit traitement de recuit dans l'étape (c) est effectuée à une température entre la température de fusion de la résine formant le composant de gaine avec une point de fusion bas et une température qui est 20 degrés (Celsius) inférieure à la température de fusion.

8. Procédé selon la revendication 7, comprenant en outre une étape consistant à agencer une pluralité des machines à fibrillation dans la direction de la machine, suivi par la formation d'un empilage des couches obtenues respectivement par des machines à fibrillation pour former un stratifié pour une traitement à une étape f) suivante comme une couche.

9. Procédé selon la revendication 8 ou 9, comprenant en outre, entre l'étape e) et l'étape f) une étape à préchauffer la couche après les fibres composites sont fondus à leurs intersections avant la couche est transportée à l'étape f).

10. Procédé selon l'une quelconque des revendications 7 à 9, dans lequel l'étape f) comprend une sous-étape de compression de la couche dans la direction d'épaisseur à l'aide d'air comprimé ou des moyens mécaniques pour augmenter la densité de la couche, et une sous-étape de fusion des fibres composites au niveau de leurs points d'intersection.

11. Procédé selon la revendication 9 ou 10, dans lequel l'étape de préchauffage décrite dans la revendication 10 est une sous-étape consistant l'émission d'un jet d'air sous pression et chauffé par une pluralité de buses individuelles qui sont disposés dans la direction transversale par rapport à la couche et qui sont transportés par les moyens de support à la direction de la machine, pour former la couche avec une pluralité de élévations et des cavités s'étendant parallèlement dans la direction de la machine, chacun des cavités est défini entre une paire d'élévations adjacentes.

12. Procédé selon l'une quelconque des revendications de 7 à 11 dans lequel l'étape e) est une sous-étape consistants l'ajouter des fibres mélangées sous forme des fibres synthétiques thermoplastiques et frisée aux fibres composites pour avoir de 0 à 50% en poids par rapport au Poids de poids totale du tissu non-tissé.

13. Procédé selon la revendication 11 ou 12, dans lequel une relation entre l'épaisseur T du tissu non-tissé mesurée dans une zone y compris une pointe d'élévation dans un plan de coupe du tissu non-tissé parallèle à la direction transversale et une largeur W mesurée à une hauteur correspondant à 1/2 de l'épaisseur T est dans une gamme de 0,55 à 1,00.

14. Procédé selon l'une quelconque des revendications 7 à 13, dans lequel l'étape b) comprend une étape consistant à alimenter le brin dans une machine de sertissage (Kräuseler) de la manière d'une boîte, de sorte que les fibres composites peuvent être serti mécaniquement en zig-zag avec 10 à 35 frisures/25 mm.

15. Procédé selon l'une quelconque des revendications 7 à 11 et 13 à 14, dans lequel lesdites fibres composites coupent une ligne verticale sous un angle aigu, notamment de 90°, par rapport au plan horizontal au plan de coupe du tissu non-tissé parallèle à la direction transversale, et certaines des fibres composites et des fibres mélangées coupent la ligne verticale sous un angle obtus supérieur à 90 degrés, de sorte qu'un angle de fibre moyenne est de 75 degrés ou moins correspondant à une valeur moyenne de l'angle de coupe aigu.

16. Procédé selon l'une quelconque des revendications 12 à 14, dans lequel lesdites fibres composites et lesdites fibres synthétiques thermoplastiques sont utilisés en tant que des fibres mélangées coupent une ligne verticale par rapport au plan horizontal sous un angle aigu, notamment de 90° au plan de coupe parallèle à la direction transversale du tissu non-tissé coupe, et certaines des fibres composites et fibres mélangées se coupent la ligne verticale sous un angle obtus supérieur à 90 degrés, de sorte qu'un angle de fibre moyenne est de 75 degrés ou moins correspondant à une valeur moyenne de l'angle de coupe aigu.

17. Procédé selon l'une quelconque des revendications 7 à 16, dans lequel l'étape e) comprend une sous-étape consistant à mélanger au moins une des fibres naturelles hydrophiles et des fibres semi-synthétiques hydrophiles dans le tissu non-tissé de sorte que le content des fibres naturelles hydrophiles ou des fibres hydrophile semi-synthétiques est 0 à 10% en poids par rapport au poids total de tissu non-tissé.

# FIG.1

# FIG.2

EP 2 161 361 B1

# FIG.3

blending fiber 112    301a    blending fiber 112    301b    blending fiber 112    301c

302a    302b    302c    302d

103

MD
TD

EP 2 161 361 B1

# FIG.4

EP 2 161 361 B1

# FIG.5

EP 2 161 361 B1

# FIG.6

FIG.7

# FIG.8A

215

215

# FIG.8B

215

215

EP 2 161 361 B1

# FIG.9

EP 2 161 361 B1

FIG.10

# FIG.11

# FIG.12

| | Fiber angle (°) |
|---|---|
| 1 | 85 |
| 2 | 79 |
| 3 | 55 |
| 4 | 87 |
| 5 | 49 |
| 6 | 80 |
| 7 | 87 |
| 8 | 78 |
| 9 | 78 |
| 10 | 84 |
| 11 | 77 |
| 12 | 64 |
| 13 | 67 |
| 14 | 65 |
| 15 | 62 |
| 16 | 65 |
| 17 | 68 |
| 18 | 70 |
| 19 | 63 |
| 20 | 64 |
| 21 | 47 |
| 22 | 74 |
| 23 | 85 |
| 24 | 65 |
| 25 | 73 |
| 26 | 75 |
| 27 | 78 |
| 28 | 80 |
| 29 | 73 |
| 30 | 54 |
| 31 | 23 |
| 32 | 60 |
| 33 | 62 |
| 34 | 42 |
| 35 | 85 |
| 36 | 83 |
| 37 | 64 |
| 38 | 74 |
| 39 | 82 |
| 40 | 80 |
| 41 | 75 |
| 42 | 84 |
| 43 | 88 |
| 44 | 79 |
| 45 | 79 |
| 46 | 79 |
| 47 | 58 |
| 48 | 68 |
| Average | 70.8 |

# FIG.13

# FIG.14

# FIG.15

| | Fiber angle (°) |
|---|---|
| 1 | 69 |
| 2 | 66 |
| 3 | 78 |
| 4 | 75 |
| 5 | 59 |
| 6 | 61 |
| 7 | 62 |
| 8 | 60 |
| 9 | 77 |
| 10 | 76 |
| 11 | 90 |
| 12 | 85 |
| 13 | 61 |
| 14 | 53 |
| 15 | 56 |
| 16 | 39 |
| 17 | 70 |
| 18 | 83 |
| 19 | 52 |
| 20 | 65 |
| 21 | 86 |
| 22 | 71 |
| 23 | 62 |
| 24 | 57 |
| 25 | 53 |
| 26 | 49 |
| 27 | 43 |
| 28 | 63 |
| 29 | 79 |
| 30 | 67 |
| 31 | 86 |
| 32 | 85 |
| 33 | 84 |
| 34 | 71 |
| Average | 67.4 |

## FIG.16

# FIG.17

# FIG.18

| | Fiber angle (° ) |
|---|---|
| 1 | 55 |
| 2 | 59 |
| 3 | 78 |
| 4 | 78 |
| 5 | 78 |
| 6 | 83 |
| 7 | 83 |
| 8 | 85 |
| 9 | 85 |
| 10 | 87 |
| 11 | 78 |
| 12 | 80 |
| 13 | 82 |
| 14 | 85 |
| 15 | 80 |
| Average | 78.4 |

# FIG.19

FIG.20

◆ Comparature Ex.1
□ Ex.1
△ Ex.2
○ Ex.13
⬤ Ex.14

Temperature changing rate (°C/sec)

0.90 0.80 0.70 0.60 0.50 0.40 0.30 0.20 0.10 0.00

Time elapsed

0  20  40  60

# FIG.21

Specification of samples

| Sample No. | Nonweven fabric | Component fiber | Nozzle pitch | Air blast rate | Conveyance velocity ratio | T/W | Average fiber angle |
|---|---|---|---|---|---|---|---|
| EXAMPLE3 | EXAMPLE3 | | 5mm | 200m/sec | 105 | 0.72 | 66.9 |
| EXAMPLE13 | | same as EXAMPLE3 | 5mm | 150m/sec | 105 | 0.70 | 69.4 |
| EXAMPLE14 | | same as EXAMPLE3 | 5mm | 150m/sec | 120 | 0.71 | 71.6 |
| EXAMPLE15 | | same as EXAMPLE3 | 5mm | 70m/sec | 105 | 0.59 | 73.4 |
| EXAMPLE16 | | same as EXAMPLE3 | 4mm | 150m/sec | 105 | 0.88 | 63.7 |
| EXAMPLE17 | | same as EXAMPLE3 | 4mm | 70m/sec | 105 | 0.72 | 70.7 |
| EXAMPLE18 | | same as EXAMPLE3 | 4mm | 150m/sec | 120 | 0.80 | 67.0 |

EP 2 161 361 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10005275 A